(19) Europäisches Patentamt
European Patent Office
Office européen des brevets

(11) **EP 4 621 454 A1**

(12) **EUROPÄISCHE PATENTANMELDUNG**

(43) Veröffentlichungstag:
**24.09.2025 Patentblatt 2025/39**

(21) Anmeldenummer: **24164836.9**

(22) Anmeldetag: **20.03.2024**

(51) Internationale Patentklassifikation (IPC):
*G02B 6/06* (2006.01)

(52) Gemeinsame Patentklassifikation (CPC):
**G02B 6/06; A61B 1/00117; A61B 1/00167;
A61B 1/0661; A61B 1/07**

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**GE KH MA MD TN**

(71) Anmelder: **Technische Universität Dresden,
Körperschaft des
öffentlichen Rechts
01069 Dresden (DE)**

(72) Erfinder:
• **KUSCHMIERZ, Robert
01069 Dresden (DE)**
• **CZARSKE, Jürgen
01069 Dresden (DE)**
• **SCHARF, Elias
01069 Dresden (DE)**
• **KROLL, Martin
01069 Dresden (DE)**

(74) Vertreter: **Kailuweit & Uhlemann Patentanwälte
Partnerschaft mbB
Bamberger Straße 49
01187 Dresden (DE)**

(54) **VERFAHREN ZUR KOMPENSATION DER LAUFZEITUNTERSCHIEDE VON BILDWELLENLEITERN**

(57) Die Erfindung betrifft ein Verfahren und eine Vorrichtung zur Kompensation der Laufzeitunterschiede von Bildwellenleitern und/oder zur Implementation eines gewünschten Laufzeitprofils, sowie die Verwendung des Verfahrens und der Vorrichtung. Das Verfahren umfasst eine Änderung der effektiven Brechungsindizes von optischen Fasern mittels hochenergetischer elektromagnetischer Strahlung, die von einem Bildwellenleiter umfasst werden. Mögliche Verwendungsbereiche des Verfahrens und der Vorrichtung sind unter anderem, jedoch nicht ausschließlich, die Krebsdiagnostik, die nichtlinearen Endomikroskopie, die optische Kohärenztomographie (*optical coherence tomography* [OCT]), die optische Kohärenztomographie mit durchgestimmter Wellenlänge der Strahlungsquelle (*swept source* OCT), die ungestörte Übertragung von Femtosekunden-Pulsen und/oder die Korrektur von Laufzeitunterschieden, die in Bildwellenleitern auftreten, welche miteinander verdrillte optische Fasern aufweisen.

Fig. 8

EP 4 621 454 A1

**Beschreibung**

Die Erfindung betrifft ein Verfahren und eine Vorrichtung zur Kompensation der Laufzeitunterschiede von Bildwellenleitern und/oder zur Implementation eines gewünschten Laufzeitprofils, sowie die Verwendung des Verfahrens und der Vorrichtung. Das Verfahren umfasst eine Änderung der effektiven Brechungsindizes von optischen Fasern mittels hochenergetischer elektromagnetischer Strahlung, die von einem Bildwellenleiter umfasst werden. Mögliche Verwendungsbereiche des Verfahrens und der Vorrichtung sind unter anderem, jedoch nicht ausschließlich, die Krebsdiagnostik, die nichtlinearen Endomikroskopie, die optische Kohärenztomographie (*optical coherence tomography* [OCT]), die optische Kohärenztomographie mit durchgestimmter Wellenlänge der Strahlungsquelle (*swept source* OCT), die ungestörte Übertragung von Femtosekunden-Pulsen und/oder die Korrektur von Laufzeitunterschieden, die in Bildwellenleitern auftreten, welche miteinander verdrillte optische Fasern aufweisen.

Endoskope zur Bildgebung und Beleuchtung werden in der Medizintechnik zur minimalinvasiven Diagnostik in schwer zugänglichen Bereichen verwendet, weshalb es zweckmäßig ist, deren Durchmesser so gering wie möglich (die Zielgröße ist unter 0,5 mm) und deren mechanische Flexibilität so hoch wie möglich zu halten. Außerdem wird von ihnen ein hoher Kontrast, sowie eine hohe Ortsauflösung und Zuverlässigkeit gefordert, wie auch geeignete optische Bildgebungsmodalitäten und geringe Kosten. Insbesondere in nichtlinearen Abbildungsverfahren, wie der nichtlinearen Endomikroskopie, sind Lichtpulse hoher Pulsleistungsdichte vonnöten, was Bildwellenleiter mit einem in der Zeitdomäne möglichst kurzen Laufzeitprofil voraussetzt. Mit Laufzeitprofil wird hier die Menge aller Laufzeitunterschiede zwischen den Laufzeiten der optischen Fasern eines Bildwellenleiters und einer Referenzlaufzeit bezeichnet. Die Referenzlaufzeit kann dabei der Mittelwert oder der Median aller Laufzeiten mehrerer optischer Fasern des Bildwellenleiters, die Laufzeit einer beliebigen Faser, oder die Laufzeit eines externen Signals sein. Andere Referenzlaufzeiten sind nicht ausgeschlossen.

Aus dem Stand der Technik sind Boroskop-Endoskope bekannt, welche auf Stab- und Gradienten-Index-Linsen (GRIN-Linsen - solche, deren Brechungsindex sich in Funktion des Abstandes vom Mittelpunkt der Linse ändert) basieren und zweidimensionale Abbildungen der Intensität elektromagnetischer Strahlung vom distalen Ende (der Anwendungsseite) zum proximalen Ende (der Instrumentenseite) liefern. Solche Endoskope weisen funktionsbedingt starre Lichtwellenleiteranordnungen mit Durchmessern von über 1 mm auf. Dadurch werden Anwendungen wie beispielsweise in der Neurochirurgie ausgeschlossen.

Außerdem schließt der Stand der Technik Kamera-Endoskope ein. Diese weisen eine hohe Flexibilität auf, da sich die Kamera und eine Beleuchtungseinheit am distalen Ende befinden und nur elektrische Signale zum proximalen Ende übertragen werden müssen. Der minimale Endoskopdurchmesser beträgt dabei 2 mm. Kamera-Endoskope erlauben ebenfalls zweidimensionale Bildgebung und keine flexible Beleuchtung. Dreidimensionale Bildgebung wird durch Stereokamerasysteme ermöglicht, erfordert aber einen höheren Endoskopdurchmesser von etwa 10 mm. Des Weiteren kann die elektromagnetische Verträglichkeit von Kamera-Endoskopen mangelhaft sein.

Bei der nichtlinearen Endomikroskopie kommen in der Regel Monomode-Lichtwellenleiter zum Einsatz. Monomode-Lichtwellenleiter weisen nur einen örtlichen Übertragungskanal auf, weshalb sie am distalen Ende komplexe 2D/3D Scanoptiken benötigen. Dadurch ist der Minimaldurchmesser auf mehrere Millimeter begrenzt. Die Scanoptiken haben hinsichtlich Bildfelddurchmesser und Wellenlänge einen beschränkten Anwendungsbereich und sind mit einem hohen Kostenaufwand verbunden. Konventionelle Endoskope weisen kohärente Bündel optischer Fasern - auch bekannt unter dem Begriff *coherent fiber bundles* (CFB) - auf, welche etwa 10.000 bis 100.000 Faserkerne enthalten. Ein geordnetes Faserbündel wird als "kohärent" bezeichnet, wenn die Positionsbeziehung zwischen jeweils zwei Fasern des Bündels über die gesamte Länge des Bündels erhalten bleibt. Solche Endoskope erlauben eine ungestörte Übertragung der zweidimensionalen Intensitätsverteilung in der Ebene der distalen Faserendfläche. Ebenen des Inspektionsgebiets können durch Integration starrer, makroskopischer Abbildungsoptiken auf die distale Faserendfläche abgebildet werden. Die relative örtliche Auflösung wird durch die Anzahl der Faserkerne bestimmt. Distale Abbildungsoptiken können die absolute örtliche Auflösung erhöhen, reduzieren aber den Bildfelddurchmesser. Der minimale Endoskopdurchmesser ist durch die notwendigen distalen Abbildungsoptiken auf den Millimeterbereich begrenzt.

CFB-Endoskope ohne komplexe Abbildungsoptiken im distalen Messkopf würden einen Endoskopdurchmesser von unter 500 μm ermöglichen, da diese nur noch durch den Faserdurchmesser begrenzt wären. Trifft eine ebenen Welle elektromagnetischer Strahlung auf ein Ende eines CFB, kann die Strahlung bei Austritt aus jeder Faser am anderen Ende des CFB eine unterschiedliche Laufzeit und eine unterschiedliche Phase aufweisen. Dies liegt an der Streuung der Materialparameter, wie beispielsweise des effektiven Brechungsindex der einzelnen Fasern. Effektive Brechungsindizes sind in der Regel wellenlängenabhängig. Die Differenz der Laufzeit zwischen der Strahlung, die aus einer Faser am anderen Ende des CFB austritt und einer Referenzlaufzeit oder der über alle Fasern gemittelten Laufzeit der austretenden Strahlung wird Laufzeitunterschied genannt. Die Menge der Laufzeitunterschiede aller Fasern des CFB wird Laufzeitprofil des CFB genannt. Es können auch Laufzeitprofile von Teilmengen aller Fasern des CFB definiert werden. Die Streuung der Laufzeit der Fasern eines CFB verhindert die ungestörte Übertragung von Femtosekundenpulsen und weitet diese in der Zeitdomäne auf. Da Laufzeiten von Fasern proportional zur optischen Weglänge sind, sind die Längen von CFB für die Verwendung in der der 2-Photonen-Mikroskopie, oder der 2-Photonen-Ablation auf etwa 10 cm begrenzt. In der Medizin werden jedoch oft faseroptische Endoskope mit Längen von mehreren Metern benötigt, wie beispielsweise in Untersuchungsverfahren von Gehirnen, die auf der Kernspinresonanzbildgebung beruhen.

Die Phasendifferenz zwischen der Strahlung, die aus einer Faser am anderen Ende des CFB austritt und der über alle Fasern gemittelten Phase der austretenden Strahlung wird Phasenstörung genannt. Die Menge der Phasenstörungen aller Fasern des CFB wird Phasenstörungsprofil genannt. Jedes CFB kann ein unterschiedliches Laufzeitprofil und ein unterschiedliches Phasenstörungsprofil aufweisen, weshalb die zeitliche Auflösung eines Signals verringert wird und die Phaseninformation der elektromagnetischen Strahlung verloren geht. Somit sind nur zweidimensionale Aufnahmen mit fester Bildebene möglich. Für eine dreidimensionale Bildgebung mit hoher Auflösung wird am häufigsten der Ansatz untersucht, die Laufzeitunterschiede Phasenstörungen der Fasern eines CFB zu messen und durch eine digitale optische Phasenkonjugation mittels programmierbarer, digitaler, optischer Flächenlichtmodulatoren - auch bekannt unter dem Begriff *spatial light modulators* (SLM) - zu kompensieren. Flächenlichtmodulatoren sind adaptive Elemente, die die Phasenmodulation elektromagnetischer Strahlung erlauben. Beispielsweise können sie Anordnungen separat ansteuerbarer, absenkbarer, anhebbarer und/oder kippbarer Mikrospiegel umfassen. Flächenlichtmodulatoren können auch als Flüssigkristalle auf einem Siliziumsubstrat - auch bekannt unter dem Begriff *liquid crystal on silicon* (LCoS) - ausgebildet sein. Durch Anlegen einer elektrischen Spannung an einzelne Kristalle eines LCoS kann deren Brechungsindex geändert werden. LCoS können ausgelegt sein, elektromagnetische Strahlung zu transmittieren und/oder zu reflektieren. Nachteilig an Flächenlichtmodulatoren ist, dass sie eine geringe Photoneneffizienz und Robustheit aufweisen, sowie teuer und justageaufwändig sind.

Ein Verfahren, bei dem die Änderung der effektiven Brechungsindizes optischer Fasern mittels hochenergetischer elektromagnetischer Strahlung implementiert wird, ist die Herstellung sogenannter Faser-Bragg-Gitter. Dies sind entlang der Länge einer optischen Faser periodisch vorkommende Stellen, deren effektiver Brechungsindex ein anderer ist als der des Rests der Faser. In eine, ein Faser-Bragg-Gitter aufweisende optische Faser eingekoppeltes Licht, dessen Wellenlänge der doppelten Gitterperiode multipliziert mit dem effektiven Brechungsindex annähernd gleicht, wird an jedem Gitterelement teilweise reflektiert. Das Herstellungsverfahren eines solchen Gitters umfasst das längsseitige Beleuchten von Abschnitten der Faser in regelmäßigen Abständen mit UV-Licht, welches geeignet ist, den effektiven Brechungsindex des Fasermaterials zu ändern. Aufgrund der benötigten längsseitigen Beleuchtung von Fasern ist das Herstellungsverfahren nicht dazu geeignet, die optischen Eigenschaften eines Lichtwellenleiters nach seiner Fertigung zu verändern. Darüber hinaus eignen sich Faser-Bragg-Gitter zur Filterung einzelner Wellenlängen, nicht jedoch zur Kompensation von Laufzeitunterschieden oder zu einer sonstigen präzisen, gezielten Änderung des Laufzeitprofils eines Lichtwellenleiters.

Aus der Druckschrift Yoshinari Maezono *et al.* geht hervor, dass der Brechungsindex von Germanium-dotierten Siliziumdioxidfasern eine höhere Fotosensitivität gegenüber UV-Strahlung der Wellenlängen 172 nm und 146 nm aufweisen, wenn sie zuvor mit Wasserstoff beladen wurden. Die Strahlung wurde zur Erstellung von Faser-Bragg-Gittern jeweils von $Xe_2{}^*$- bzw. $Kr_2{}^*$-Excimerlampen erzeugt. Nachteilig ist die Methode zwar geeignet, Strahlung einzelner Wellenlängen effektiver während der Übertragung innerhalb optischer Fasern herauszufiltern, doch nicht, um die Laufzeit der Strahlung gezielt zu kontrollieren. Ebenfalls lassen sich die Faser-Bragg-Gitter nach Fertigung eines Lichtwellenleiters samt äußerer Hülle nicht nachträglich erstellen oder verändern.

In Lancry *et al.* wird der Zusammenhang zwischen der chemischen Zusammensetzung von Preformen optischer Fasern und Schwellenwerten der Pulsenergien von Femtosekundenlasern beschrieben, welche Änderungen der Brechungsindizes der Preformen hervorrufen, die aus dotierten Siliziumdioxidgläsern bestehen. Nachteilig geht aus der Druckschrift nicht hervor, wie der Effekt für Bildwellenleiter verwendet werden kann, um deren Laufzeiten, bzw. Laufzeitprofile zu gezielt zu ändern.

Die Druckschrift US 2021/0382290 A1 offenbart eine Vorrichtung zum Transport und zur Steuerung von Lichtstrahlen, die einen Lichtwellenleiter umfasst, der ein Bündel optischer Monomode-Fasern aufweist, wobei jede optische Monomode-Faser dazu bestimmt ist, einen Lichtstrahl an einem proximalen Ende zu empfangen und einen Lichtstrahl an einem distalen Ende zu emittieren, wobei das Bündel von optischen Monomode-Fasern im Betrieb einen minimalen Krümmungsradius aufweist, der einer maximalen Krümmung des Faserbündels entspricht. Die Vorrichtung umfasst außerdem einen SLM zur Phasensteuerung, der auf der Seite des proximalen Endes des Lichtwellenleiters angeordnet ist und der geeignet ist, jeden der am proximalen Ende zu empfangenen Lichtstrahlen mit einer Phasenverschiebung zu beaufschlagen, um am distalen Ende des Lichtwellenleiters einen Beleuchtungsstrahl mit einer vorbestimmten Phasenfunktion zu bilden. Das Bündel optischer Monomode-Fasern ist verdrillt und weist eine Verdrillungsperiode auf, die dazu geeignet ist, die Phasenfunktion und das Laufzeitprofil des Bündels am distalen Ende des Lichtwellenleiters aufrechtzuerhalten, wenn das Bündel optischer Monomode-Fasern einer Krümmung unterliegt, die geringer ist als die maximale Krümmung. Die Vorrichtung ist dazu geeignet, optische Pulse mit einer Pulsdauer zwischen 100 fs und 10 ns zu führen. Nachteilig ist, dass die optischen Fasern des Bündels aufgrund der Verdrillung zusätzliche Laufzeitunterschiede aufweisen, auch wenn diese bei Biegung des Bündels konstant bleiben.

In der Druckschrift US 2022/0248938 A1 werden ein optisches System und ein Bildgebungsverfahren offenbart. Das optische System weist dabei einen aus mehreren optischen Fasern bestehenden Multifaserleiter und einen optischen Diffusor auf, der es erlaubt, ein Intensitätsmuster auf den Multifaserleiter abzubilden. Das Intensitätsmuster repräsentiert Phaseninformationen von Licht, welches von mindestens einem dreidimensionalen Objekt ausgesendet wird. Der Wellenleiter ist dazu eingerichtet, das Intensitätsmusters in Form einer Vielzahl von Bildpunkten zu einem Auswertesystem zu übertragen. Dabei ist das Auswertesystem eingerichtet, ein Bild des Objektes zu erzeugen, wobei die Erzeugung auf dem mittels des Wellenleiters übertragenen Intensitätsmuster basiert. Nachteilig ist, die komplexwertige Übertragungsfunktion des Systems nicht definiert werden kann und die 3D-Bildgebung ausschließlich aus Intensitätsinformation gewonnen werden muss.

Die Druckschrift Mirsky & Shaked stellt ein System vor, um die Einschränkung des Sichtfeldes in der Off-Axis-Holografie zu überwinden. Dabei wird die Nutzung eines Mach-Zehnder-Interferometern zum Off-Axis-Multiplexing erwähnt. Nachteilig eignet sich das System nicht zur Kompensation der Laufzeitunterschieden von Bildwellenleitern.

Aufgabe der Erfindung ist es daher, ein Verfahren und eine Vorrichtung bereitzustellen, welche die Nachteile des Standes der Technik überwinden, indem sie es ermöglichen, die Laufzeitunterschiede zwischen optischen Fasern von Bildwellenleitern zu verkürzen, bzw. Bildwellenleiter mit einem bestimmten Laufzeitprofil zu beaufschlagen.

Erfindungsgemäß wird die Aufgabe durch ein Verfahren, eine Vorrichtung sowie Verwendungen gemäß den unabhängigen Ansprüchen gelöst. Vorteilhafte Ausgestaltungen der Erfindung sind in den abhängigen Ansprüchen angegeben.

Ein Aspekt der Erfindung betrifft ein Verfahren zur Kompensation von Laufzeitunterschieden und/oder zur Implementation eines gewünschten Laufzeitprofils zumindest eines wenigstens zwei optische Fasern aufweisenden Bildwellenleiters, umfassend die Schritte

- Bereitstellen zumindest eines wenigstens zwei optische Fasern aufweisenden Bildwellenleiters,
- Auswählen einer ersten Teilmenge zumindest zweier optischer Fasern des Bildwellenleiters und Vermessen der Laufzeitunterschiede der optischen Fasern der ersten Teilmenge in mindestens einer elektromagnetischen Wellenlänge,
- Auswählen einer zweiten Teilmenge einer oder mehrerer optischer Fasern und einer dritten Teilmenge mindestens zweier optischer Fasern des Bildwellenleiters, Änderung des effektiven Brechungsindizes jeder der optischen Fasern der zweiten Teilmenge durch longitudinale Einkopplung hochenergetischer elektromagnetischer Strahlung in jede der optischen Fasern der zweiten Teilmenge an einem ersten Ende und/oder einem zweiten Ende des Bildwellenleiters, so, dass die Laufzeitunterschiede der dritten Teilmenge optischer Fasern beliebig reduziert sind und/oder so, dass sich die Laufzeitunterschiede der dritten Teilmenge optischer Fasern dem Wert des gewünschten Laufzeitprofils beliebig annähern,

wobei die zweite Teilmenge optischer Fasern mindestens eine optische Faser der ersten Teilmenge umfasst und die dritte Teilmenge optischer Fasern mindestens eine optische Faser der zweiten Teilmenge umfasst und dabei die erste Teilmenge optischer Fasern diese mindestens eine optische Faser der zweiten Teilmenge ebenfalls umfasst, wobei die Durchführung des Schrittfolge ii) - iii) entweder einmalig erfolgt oder so oft erfolgt, bis eine gewünschte Kompensation der Laufzeitunterschiede und/oder das gewünschte Laufzeitprofil des zumindest einen Bildwellenleiters in der mindestens einen Wellenlänge implementiert ist, wobei bei wiederholten Durchführungen der Schrittfolge ii) - iii) die Teilmengen optischer Fasern entweder jeweils den jeweiligen Teilmengen optischer Fasern der vorherigen Durchführung der Schrittfolge ii) - iii) gleichen oder neu ausgewählt werden und wobei die Schritte ii) und iii) sequenziell oder gleichzeitig erfolgen.

In Ausführungsformen des Verfahrens umfassen

- die erste Teilmenge optischer Fasern alle optischen Fasern des Bildwellenleiters und/oder
- die zweite Teilmenge optischer Fasern alle optischen Fasern der ersten Teilmenge mit Ausnahme derer, deren Laufzeitunterschied zur Referenzlaufzeit bereits ausreichend gering ist und/oder bereits dem gewünschten Laufzeitprofil entspricht und/oder
- die dritte Teilmenge optischer Fasern alle optischen Fasern der ersten Teilmenge.

Die Laufzeitunterschiede der optischen Fasern der ersten Teilmenge zur Referenzlaufzeit werden in Ausführungsformen des Verfahrens einzeln vermessen.

In Ausführungsformen des Verfahrens wird die hochenergetische Strahlung einzeln in die Fasern der zweiten Teilmenge eingekoppelt. In der Regel weist jede optische Faser des Bildwellenleiters eine andere Laufzeit auf, weshalb jede optische Faser eine andere Änderung des effektiven Brechungsindex benötigt, um die Laufzeitunterschiede ausreichend zu kompensieren, bzw. das gewünschte Laufzeitprofil zu erreichen. Aus diesem Grund wird die hochenergetische elektromagnetische Strahlung in jede optische Faser einzeln eingekoppelt und dabei jeweils mit unterschiedlichen Eigenschaften beaufschlagt, die jeweils zu einer Annäherung des effektiven Brechungsindizes der jeweiligen optischen Faser an den effektiven Brechungsindex führt, der für die jeweilige gewünschte Laufzeit benötigt wird.

Die Schritte ii) und iii) können gleichzeitig erfolgen, wenn das Vermessen der Laufzeitunterschiede der optischen Fasern mit derselben hochenergetischen elektromagnetischen Strahlung erfolgt, die geeignet ist, den effektiven Brechungsindex der optischen Fasern zu ändern.

In Ausführungsformen des Verfahrens umfasst die hochenergetische elektromagnetische Strahlung ultra-kurze Pulse und/oder UV-Strahlung, insbesondere Femtosekunden-Laserpulse und/oder Excimer-Licht, wobei das Excimer-Licht bevorzugt 146-nm-Excimer-Licht oder 248-nm Excimer-Licht beinhaltet und wobei das Excimer-Licht von Excimerlampen emittierbares Excimer-Licht und/oder Excimer-Laserlicht umfasst.

Die Pulsdauer der ultra-kurzen Pulse kann in Ausführungsformen zwischen 10 fs und 10 ps liegen und die Pulsdauer der Femtosekunden-Laserpulse kann zwischen 10 fs bis 1 ps liegen.

Der Begriff "Licht", umfasst, auch als Wortteil, das elektromagnetische Spektrum mit Wellenlängen zwischen einschließlich 100 nm und einschließlich 10 $\mu$m.

Die optischen Fasern, für die das erfindungsgemäße Verfahren durchgeführt wird, sind bevorzugt Monomode-Fasern. Die Kerne der optischen Fasern haben bevorzugt einen Durchmesser, der klein genug ist, höchstens eine einzelne Mode elektromagnetischer Strahlung, die zum Vermessen der Laufzeitunterschiede des Bildwellenleiters verwendet wird und/oder die zur Änderung der effektiven Brechungsindizes der optischen Fasern verwendet wird, zu transmittieren, aber groß genug ist, dass ein signifikantes Übersprechen > 3dB auf benachbarte optische Fasern verhindert wird. Das bedeutet, dass der Durchmesser der optischen Fasern in derselben Größenordnung liegt, wie der Durchmesser der Mode der elektromagnetischen Strahlung, die zum Vermessen der Laufzeitunterschiede des Bildwellenleiters verwendet wird und/oder die zur Änderung der effektiven Brechungsindizes der optischen Fasern verwendet wird. Besonders bevorzugt ist der Durchmesser der Kerne der optischen Fasern kleiner als der doppelte Durchmesser der Mode und größer als ein Fünftel des Durchmessers der Mode der elektromagnetischen Strahlung, die zum Vermessen der Laufzeitunterschiede des Bildwellenleiters verwendet wird und/oder die zur Änderung der effektiven Brechungsindizes der optischen Fasern verwendet wird.

Eine Änderung des effektiven Brechungsindex jeder der ausgewählten optischen Fasern der zweiten Teilmenge ausgewählter optischer Fasern wird in Ausführungsformen des Verfahrens dadurch erreicht, dass eine Modulation einer oder mehrerer Stellgrößen der hochenergetischen elektromagnetischen Strahlung, ausgewählt aus der Leistung, der Energie, der Pulsdauer, der Pulsform, dem spektralen Bereich, dem spektralen Verlauf der Leistung, dem zeitlichen Verlauf der Leistung, dem spektralen Verlauf der Energie, dem zeitlichen Verlauf der Energie und der Polarisation erfolgt.

Beispielsweise kann der effektive Brechungsindex einer optischen Faser durch Einkopplung in die optische Faser von kontinuierlich emittiertem UV-Excimer-Licht

- mit geringer Leistung über einen langen Zeitraum hinweg, oder
- mit hoher Leistung über einen kurzen Zeitraum hinweg

geändert werden. Es ist beispielsweise auch möglich, den effektiven Brechungsindex einer optischen Faser durch Einkopplung in die optische Faser von gepulstem IR-Laserlicht oder gepulstem sichtbarem Laserlicht mit einer Pulsleistung von 10 MW zu ändern.

In Ausführungsformen des Verfahrens

- ist der zumindest eine Bildwellenleiter vor und/oder während der Durchführung der Schrittfolge ii)-iii) einer $H_2$ und/oder $N_2$ umfassenden Atmosphäre ausgesetzt, um den $H_2$- und/oder $N_2$-Partialdruck im inneren des Bildwellenleiters zu erhöhen und/oder
- erfolgt das Auswählen der Modulation des zeitlichen und spektralen Verlaufs der Strahlungsleistung von Pulsen der ultra-kurze Pulse aufweisenden hochenergetischen elektromagnetischen Strahlung vorteilhaft so, dass die über das gesamte Spektrum integrierte Strahlungsleistung bei einem ausgewählten Abstand vom ersten Ende des Bildwellenleiters innerhalb mindestens einer der ausgewählten optischen Fasern einen Maximalwert annimmt und die Modulation des zeitlichen und spektralen Verlaufs der Strahlungsleistung der hochenergetischen elektromagnetischen Strahlung bei jeweils wiederholter Durchführung der Schrittfolge ii) - iii) besonders vorteilhaft so ausgewählt ist, dass bei jeder Wiederholung die Strahlungsleistung bei einem anderen als den bei der vorherigen Durchführung der Schrittfolge ii) - iii) ausgewählten Abstand einen Maximalwert annimmt.

Dadurch, dass im Bildwellenleiter der Partialdruck von $H_2$ und/oder $N_2$ gegenüber dem der Erdatmosphäre erhöht wird, ist es möglich, durch die Einkopplung hochenergetischer elektromagnetischer Strahlung einer bestimmten Leistung, Energie, eines bestimmten spektralen Bereichs, spektralen Verlaufs der Leistung, zeitlichen Verlaufs der Leistung, spektralen Verlaufs der Energie, zeitlichen Verlaufs der Energie und der Polarisation in optische Fasern eines Bildwellenleiters eine größere Änderung des effektiven Brechungsindizes der optischen Fasern zu erzielen, als es bei einem identischen Bildleiter der Fall wäre, dessen Partialdruck von $H_2$ und/oder $N_2$ nicht erhöht ist.

Eine räumliche Änderung des Brechungsindex innerhalb eines elektromagnetischen Feldes, wie es an einer Grenzfläche zwischen zwei Materialien mit unterschiedlichen Brechungsindizes der Fall ist, führt dazu, dass an dieser Grenzfläche elektromagnetische Energie absorbiert wird. Wenn kontinuierlich emittierte hochenergetische elektromagnetische Strahlung an einem Ende in optische Fasern eines Bildwellenleiters eingekoppelt werden, wird an diesem Ende der optischen Faser mehr Energie absorbiert als im Rest der Faser. Dies führt zunächst zu einer größeren Änderung des effektiven Brechungsindizes, als im Rest der Faser und kann nach andauernder oder wiederholter Einkopplung der hochenergetischen elektromagnetischen Strahlung zu einem Schaden der optischen Faser im Bereich desselben Endes führen. Da der Brechungsindex eines Mediums wellenlängenabhängig ist und umgekehrt proportional zur Ausbreitungsgeschwindigkeit elektromagnetischer Strahlung innerhalb des Mediums ist, ist die Ausbreitungsgeschwindigkeit von der Wellenlänge abhängig.

Wenn die Ausbreitungsgeschwindigkeit elektromagnetischer Strahlung in einem Medium mit steigender Wellenlänge strikt monoton steigt, würde sich ein breitbandiger Puls elektromagnetischer Strahlung innerhalb des Mediums so ausbreiten, dass ein langwelligerer Teil des Pulses das Mediums als erstes durchquert, gefolgt von einem kurzwelligen Teil des Pulses. Falls ein Puls jedoch so geformt wird, dass kurzwellige Strahlung als erste in das Medium eingekoppelt wird, gefolgt von langwelligerer Strahlung, ist es möglich, dass die kurzwellige und langwellige Strahlung einen Bereich innerhalb des Mediums gleichzeitig erreichen und somit die Pulsleistung in diesem Bereich ein Maximum erreicht und nicht am Grenzflächenbereich, an dem die Strahlung in das Medium eingekoppelt wurde. Durch Verringerung, bzw. Vergrößerung der Verzögerung zwischen kurz- und langwelliger Strahlung der emittierten Pulse kann jeweils ein vom Grenzflächenbereich näherer, bzw. entfernterer Bereich ausgewählt werden, an dem die Pulsleistung maximiert wird. Somit kann verhindert werden, dass bei wiederholter Einkopplung ultrakurzer elektromagnetischer Pulse die Strahlungsleistung stets im selben Bereich eines festen Mediums ihren Maximalwert erreicht und in diesem Bereich Schäden am Medium verursacht.

Durch ultrakurze Pulse hochenergetischer elektromagnetischer Strahlung verursachbare Schäden des Bildwellenleiters im Bereich des ersten Endes und/oder des zweiten Endes werden in Ausführungsformen des Verfahrens gering gehhalten

- durch Verringern der Differenz zwischen den effektiven Brechungsindizes der Fasern und des an dem ersten Ende und/oder dem zweiten Ende des Bildwellenleiters angrenzenden Mediums während der Durchführung des Verfahrens durch

    ◦ Umgeben des ersten Endes und/oder dem zweiten Ende des Bildwellenleiters mit einer Immersionsflüssigkeit, bevorzugt einem Immersionsöl und/oder
    ◦ Bringen in Berührungskontakt zumindest einer Glasplatte mit dem ersten Ende und/oder dem zweiten Ende des Bildwellenleiters,
    wobei die Materialien, aus dem die Immersionsflüssigkeit und/oder die Glasplatte bestehen, mindestens jeweils ein Material umfassen, dessen Brechungsindex dem effektiven Brechungsindex mindestens einer optischen Faser der zweiten Teilmenge ausgewählter optischer Fasern beliebig nah ist und/oder

- durch Entfernen eines Teils des zumindest einen Bildwellenleiters entlang einer Ebene am ersten Ende und/oder am zweiten Ende nach der Durchführung von Schritt iii), wobei die Ebene senkrecht zur optischen Achse des Bildwellenleiters ist und die Länge des zu entfernenden Teils oder der zu entfernenden Teile des Bildwellenleiters entlang der optischen Achse der Länge des Teils oder der Teile des Bildwellenleiters entspricht oder entsprechen, der oder die durch die longitudinale Einkopplung hochenergetischer elektromagnetischer Strahlung zerstört wurde oder wurden und/oder
- durch ein Aufweiten der Kerne der optischen Fasern am ersten Ende und/oder am zweiten Ende des Bildwellenleiters.

Wenn der Unterschied zwischen den Brechungsindizes der optischen Fasern und dem umgebenden Medium gering ist, ist auch die Absorption der Energie durch die optischen Fasern an der Grenzfläche zwischen den optischen Fasern und dem umgebenden Medium gering im Vergleich zur Absorption der Energie durch die optischen Fasern an der Grenzfläche, wenn das umgebende Medium Luft bei Standardbedingungen ist.

Wenn der Kern einer optischen Faser an einem der Enden des Bildwellenleiters aufgeweitet ist, ist die Flächenleistungsdichte der in den Kern der optischen Faser eingekoppelten hochenergetischen elektromagnetischen Strahlung eine geringere, als sie die Flächenleistungsdichte der gleichen Strahlung in eine optische Faser, deren Kern an keinem der Enden des Bildwellenleiters aufgeweitet ist. Durch das Aufweiten der Kerne der optischen Fasern am ersten Ende und/oder am zweiten Ende des Bildwellenleiters verringert sich so der Beschädigung, bzw. die Ablation, die das Einkoppeln der hochenergetischen elektromagnetischen Strahlung verursacht. Das Aufweiten einer optischen Faser an einem Ende des Bildwellenleiters kann durch ein Aufheizen erreicht werden, welches Dotierstoffe im Kern der optischen Faser in den Mantel der optischen Faser diffundieren lässt, wodurch das Brechungsindexprofil der optischen Faser am aufgeheizten Ende des Bildwellenleiters verschmiert wird.

Ein Teil eines Bildwellenleiters gilt als beschädigt, wenn die Transmission im Wellenlängenbereich, der zur Vermessung der Laufzeitunterschiede verwendet wird, so stark gesunken ist, dass der Bildwellenleiter nicht mehr für den gewünschten Zweck verwendbar ist.

In Ausführungsformen des Verfahrens erfolgt das Vermessen des Laufzeitunterschiedes mittels Weißlichtinterferometrie und/oder OCT und/oder Mehrwellenlängenholographie.

In weiteren Ausführungsformen ist die Mehrwellenlängenholographie als off-axis-Holographie unter Verwendung eines Mach-Zehnder-Interferometers ausgebildet.

In Ausführungsformen des Verfahrens wird Licht in einen Y-Wellenleiter eingekoppelt und aufgeteilt. Ein erster Teil des Lichtes wird in einen bereitgestellten Bildwellenleiter gekoppelt, vom Bildwellenleiter in ein erstes Vergrößerungsobjektiv gekoppelt, sowie aufgeweitet und gelangt zu einem Strahlteiler. Der Strahlteiler transmittiert $X\%$ des ersten Teils des Lichtes zu einem bildgebenden Detektor für elektromagnetische Strahlung. Ein zweiter Teil des Lichtes wird so aus dem Y-Wellenleiter ausgekoppelt, dass er so auf den Strahlteiler trifft, dass der Strahlteiler $X\%$ des zweiten Teils des Lichtes zu einem Spiegel transmittiert, der Spiegel die $X\%$ des zweiten Teils des Lichtes zum Strahlteiler reflektiert und der Strahlteiler $(100 - X)\%$ von den $X\%$ des zweiten Teils des Lichtes zum bildgebenden Detektor reflektiert.

Das erste Vergrößerungsobjektiv und der bildgebende Detektor sind so zueinander und zum Bildwellenleiter angeordnet und jeweils so ausgebildet, dass Strukturen, wie Interferenzmuster an den Facetten der optischen Fasern, vom bildgebenden Detektor aufgelöst werden können. Der Spiegel wird entlang der optischen Achse der $X\%$ des zweiten Teils des Lichtes bewegt, bis eine der optischen Fasern aus der Perspektive des bildgebenden Detektors ein Interferenzmuster aufweist. Die Laufzeit des Anteils des Lichtes, welches durch die das Interferenzmuster aufweisende optische Faser geleitet wird, kann als Referenzlaufzeit ausgewählt werden. Der Spiegel wird weiter entlang der optischen Achse der $X\%$ des zweiten Teils des Lichtes bewegt, bis jede optische Faser des Bildwellenleiters, von der eine Messung des Laufzeitunterschiedes bezüglich zur Referenzlaufzeit erwünscht ist, im Laufe der Bewegung des Spiegels ein Interferenzmuster aufgewiesen hat. Dabei wird bei jedem Erscheinen eines Interferenzmusters die Position des Spiegels und der das Interferenzmuster aufweisenden optischen Faser auf einem Speichermedium registriert. Anhand der relativen Position des Spiegels und der Kenntnis der Lichtgeschwindigkeit im den Spiegel umgebenden Medium, wird der Laufzeitunterschied bezüglich zur Referenzlaufzeit für jede der optischen Fasern ermittelt, welche bei einer bestimmten Position des Spiegels ein Interferenzmuster aufweisen.

X kann eine beliebige reelle Zahl im Bereich $0 < X < 100$ sein. Bevorzugt ist $X = 50$. Bevorzugt betragen der erste Teil und der zweite Teil des Lichtes jeweils 50% des in den Y-Wellenleiter eingekoppelten Teil des Lichtes. Bevorzugt umfasst das Licht von einer Superlumineszenzdiode emittiertes Licht. Weitere optische Komponenten im Strahlengang, wie Polarisationsfilter, Linsen, Strahlteiler und/oder Spiegel werden im Verfahren nicht ausgeschlossen.

Hochenergetische elektromagnetische Strahlung wird durch das erste Vergrößerungsobjektiv in einzelne optische Fasern des Bildwellenleiters eingekoppelt.

In Ausführungsformen sind das erste Vergrößerungsobjektiv, der Bildwellenleiter, sowie die Quelle hochenergetischer elektromagnetischer Strahlung so zueinander positioniert, dass die hochenergetische Strahlung in jeweils eine einzelne optische Faser des Bildwellenleiters eingekoppelt werden kann. Die relative Position der Quelle hochenergetischer elektromagnetischer Strahlung, des Bildwellenleiters und des ersten Vergrößerungsobjektives kann nach jeder erfolgten Einkopplung der hochenergetischen elektromagnetischen Strahlung in eine der optischen Fasern dabei so verändert werden, dass die hochenergetische elektromagnetische Strahlung in eine weitere optische Faser des Bildwellenleiters eingekoppelt werden kann.

Ein Flächenlichtmodulator ist in Ausführungsformen so im Strahlengang der hochenergetischen elektromagnetischen Strahlung angeordnet und so eingestellt, dass die hochenergetische Strahlung in jeweils eine einzelne optische Faser des Bildwellenleiters eingekoppelt werden kann. Die Einstellung des Flächenlichtmodulators kann nach jeder erfolgten Einkopplung der hochenergetischen elektromagnetischen Strahlung in eine der optischen Fasern dabei so verändert werden, dass die hochenergetische elektromagnetische Strahlung in eine weitere optische Faser des Bildwellenleiters eingekoppelt werden kann.

In Ausführungsformen des Verfahrens werden die funktionalen Zusammenhänge zwischen der einen oder der mehreren Stellgrößen der hochenergetischen elektromagnetischen Strahlung und Laufzeitänderungen optischer Fasern jeweils durch eine Kalibration ermittelt.

Beispielsweise können zur Kalibration die Laufzeiten und/oder die effektiven Brechungsindizes von einer oder mehreren optischen Fasern mit optischen und materiellen Eigenschaften und Längen, welche denen des Bildwellenleiters ähneln, gemessen werden und anschließend oder gleichzeitig mit hochenergetischer elektromagnetischer Strahlung mit bestimmten Stellgrößen beaufschlagt werden und die Laufzeiten und/oder die effektiven Brechungsindizes erneut gemessen werden. Dieser Vorgang kann mit jeweils einer Faser mehrfach wiederholt werden. Daraufhin können die Stellgrößen zusammen mit den dazugehörigen Änderungen der Laufzeiten und/oder effektiven Brechungsindizes in einer oder mehreren Kalibrationstabellen eingetragen werden.

Zusätzlich zu den Stellgrößen, Laufzeiten und/oder den effektiven Brechungsindizes von zur Kalibration verwendeten optischen Fasern können auch die durch die Beaufschlagung mit hochenergetischer elektromagnetischer Strahlung verursachten Änderungen der Transmission der zur Kalibration verwendeten optischen Fasern gemessen und in die Kalibrationstabelle eingetragen werden.

Andere Kalibrationsverfahren sind nicht ausgeschlossen.

In Ausführungsformen des Verfahrens erfolgt nach Durchführung von Schritt iii) ein Verfahren zur Kompensation von Phasenstörung von mindestens zwei Wellenlängen $\lambda_k$ des zumindest einen Bildwellenleiters und/oder zur Implementation mindestens einer optischen Funktion, welche Ausbreitungsrichtungen elektromagnetischer Strahlung von mindestens einer Wellenlänge $\lambda_f$ beim Ein- und/oder Austritt in den und/oder aus dem Bildwellenleiter ändert, umfassend das Modulieren der einen funktionalen Zusammenhang mit einer Referenzweglänge aufweisenden elektromagnetischen Phasenstörung $\varphi_{ist}$ einer fünften Teilmenge mindestens einer optischen Faser $j$, die ausgewählt ist aus einer vierten Teilmenge zweier oder mehrerer optischer Fasern des Bildwellenleiters, für jede der Wellenlängen $\lambda_k$ und/oder $\lambda_f$, umfassend die Teilschritte:

a) Vermessen der elektromagnetischen Phasenstörung $\varphi_{ist}$ für jede der Wellenlängen $\lambda_k$ und/oder $\lambda_f$ an den optischen Fasern der vierten Teilmenge,

b) Bestimmung einer gewünschten modulierten Phase $\varphi_{soll}$ für jede der fünften Teilmenge ausgewählter optischer Fasern $j$ und für jede der Wellenlängen $\lambda_k$ und/oder $\lambda_f$, wobei die gewünschte modulierte Phase $\varphi_{soll}$ für jede der Wellenlängen $\lambda_k$ und/oder $\lambda_f$ unabhängig voneinander bestimmt wird oder abhängig von $\varphi_{soll}$ für eine oder mehrere der anderen Wellenlängen $\lambda_k$ und/oder $\lambda_f$ bestimmt wird,

c) Ermittlung eines funktionalen Zusammenhangs zwischen einer Stellgröße $x_j$ und einer Phasenänderung $\varphi_{stell}$ für jede der Wellenlängen $\lambda_k$ und/oder $\lambda_f$ und jeder der ausgewählten optischen Fasern $j$ der fünften Teilmenge,

d) Definition einer Fehlerfunktion $f$ zur Beschreibung der Gesamtabweichung zwischen einer resultierenden Phase $\varphi_{res} = (\varphi_{ist} + \varphi_{stell})\mod(2\pi)$ und der gewünschten modulierten Phase $\varphi_{soll}$ über alle Wellenlängen $\lambda_k$ und/oder $\lambda_f$ für jede der ausgewählten optischen Fasern $j$ der fünften Teilmenge,

e) Ermittlung des Wertes $x_{j\_fmin}$ der Stellgröße $x_j$ bei der die Fehlerfunktion $f$ einen minimalen Wert annimmt für jede der ausgewählten optischen Fasern $j$ der fünften

f) Teilmenge,

- Bereitstellen und Positionieren eines Elements zur Kompensation von Phasenstörung von mindestens zwei Wellenlängen $\lambda_k$ eines Bildwellenleiters und/oder zur Implementation einer optischen Funktion, welche Ausbreitungsrichtungen elektromagnetischer Strahlung von mindestens einer Wellenlänge $\lambda_f$ beim Ein- und/oder Austritt in den und/oder aus dem Bildwellenleiter ändert, hinter dem ersten Ende und/oder hinter dem zweiten Ende des Bildwellenleiters, derart, dass das Element entlang der optischen Achse jeder der ausgewählten optischen Fasern $j$ der fünften Teilmenge den Wert $x_{j\_fmin}$ der Stellgröße $x_j$ aufweist,

und/oder

- Verkürzung und/oder Verlängerung jeder ausgewählten optischen Fasern $j$ der fünften Teilmenge zur Kompensation der Phasenstörung und/oder zur Implementation einer optischen Funktion, welche Ausbreitungsrichtungen elektromagnetischer Strahlung beim Ein- und/oder Austritt in den und/oder aus dem Bildwellenleiter ändert, am ersten Ende und/oder am zweiten Ende des Bildwellenleiters, derart, dass die Verkürzung und/oder die Verlängerung für jede der ausgewählten optischen Fasern $j$ der fünften Teilmenge und jeder der Wellenlängen $\lambda_k$ und/oder $\lambda_f$ den Wert $x_{j\_fmin}$ der Stellgröße $x_j$ aufweist,

so dass der das Element und/oder die Verkürzung und/oder Verlängerung jeder der ausgewählten optischen Fasern $j$ der fünften Teilmenge aufweisende Bildwellenleiter für jede der Wellenlängen $\lambda_k$ und/oder $\lambda_f$ und jeder der ausgewählten optischen Fasern $j$ der fünften Teilmenge eine resultierende Phase $\varphi_{res\_fmin}$ aufweist, bei der die Fehlerfunktion $f$ einen minimalen Wert annimmt.

In Ausführungsformen des Verfahrens umfassen

- die vierte Teilmenge optischer Fasern alle optischen Fasern des Bildwellenleiters und/oder

- die fünfte Teilmenge optischer Fasern alle optischen Fasern der vierten Teilmenge mit Ausnahme derer, deren Phasenstörung bereits ausreichend gering ist und/oder bereits der gewünschten optischen Funktion entsprechen.

Die Menge Wellenlängen $\lambda_k$ und die Menge der Wellenlängen $\lambda_f$ können vollkommen unterschiedlich sein, miteinander überlappen oder identisch sein.

Die Phasenstörung $\varphi_{ist}$ einer optischen Faser mit Index $j$ bei einer Wellenlänge $\lambda$, kann durch die Formel

$$\varphi_{ist}(\lambda, j) = 2\pi \left( \frac{\Delta L(\lambda, j)}{\lambda} \bmod 1 \right)$$

beschrieben werden, wobei $\Delta L$ eine Abweichung einer optischen Weglänge der optischen Faser $j$ bei der Wellenlänge $\lambda$ von der gemittelten optischen Weglänge bei der Wellenlänge $\lambda$ aller optischer Fasern der vierten Teilmenge ist.

Andere Beschreibungen der Phasenstörung $\varphi_{ist}$ sind nicht ausgeschlossen. In einer alternativen Ausführungsform kann statt $\Delta L$ eine Referenzweglänge verwendet werden, welche die Abweichung der optischen Weglänge einer optischen Faser zu einer beliebigen Referenzlänge ist.

Die gewünschte modulierte Phase $\varphi_{soll}$ kann für jede der Wellenlängen unabhängig voneinander oder abhängig von der gewünschten modulierten Phase $\varphi_{soll}$ für eine oder mehrere der anderen Wellenlängen bestimmt werden, wodurch es möglich ist, für verschiedene Wellenlängen unterschiedliche optische Funktionen zu implementieren. Das bedeutet beispielsweise, dass für elektromagnetische Strahlung einer ersten Wellenlänge eine Bündelung der Strahlung auf einen Brennpunkt gewünscht ist, während für Strahlung einer zweiten Wellenlänge eine Doughnut-Mode gewünscht ist und für Strahlung einer dritten Wellenlänge eine Kippung der Ausbreitungsrichtung gewünscht ist. Kombinationen solcher optischen Funktionen, wie beispielsweise die Kippung der Ausbreitungsrichtung und die Bündelung der Strahlung einer Wellenlänge auf einen Brennpunkt sind auch möglich. Es ist auch möglich, dass $\varphi_{soll}$ für verschiedene Wellenlängen so gewählt wird, dass die Ausbreitungsrichtung der Strahlung für jede der verschiedenen Wellenlängen um einen für jede der verschiedenen Wellenlängen unterschiedlichen Winkel gekippt wird und/oder an einem für jede der verschiedenen Wellenlängen unterschiedlichen Brennpunkt gebündelt wird. Beispiele für solche optischen Funktionen sind die Fokussierung der Strahlung auf einen Brennpunkt in einer Ebene, ähnlich wie es durch eine konvexe Linse ermöglicht wird, die Kippung der Strahlung, oder die Erzeugung einer Doughnut-Mode, also einer ringförmigen Verteilung der Intensität elektromagnetischer Strahlung in einer Ebene.

In Ausführungsformen des Verfahrens zur Kompensation von Phasenstörung und/oder zur Implementation mindestens einer optischen Funktion ist die gewünschte modulierte Phase $\varphi_{soll}$ durch die Formel $\varphi_{soll}(\lambda, j) = (\varphi_{ist}(\lambda, j) + \varphi_{hu}(\lambda, j)) \bmod (2\pi)$ beschreibbar, wobei $\varphi_{hub}(\lambda, j)$ ein gewünschter Phasenhub ist.

Eine Änderung der optischen Weglänge, welche in der gewünschten modulierten Phase $\varphi_{soll}$ resultiert, kann durch die Formel

$$L_{soll}(\lambda, j) = \frac{\lambda\, \varphi_{hub}(\lambda, j)}{2\pi} + N\,\lambda$$

beschrieben werden, wobei $N$ eine beliebige ganze Zahl ist.

In weiteren Ausführungsformen des Verfahrens zur Kompensation von Phasenstörung und/oder zur Implementation mindestens einer optischen Funktion liegt $N$ in dem Bereich zwischen einschließlich -9 und einschließlich +9.

In Ausführungsformen des Verfahrens zur Kompensation von Phasenstörung und/oder zur Implementation mindestens einer optischen Funktion, in denen es gewünscht ist, die Phasenstörung $\varphi_{ist}$ für eine Wellenlänge $\lambda$ und eine optische Faser $j$ zu kompensieren und auch die Implementation einer zusätzlichen optischen Funktion $\varphi_{zus}$ gewünscht ist, kann der gewünschte Phasenhub $\varphi_{hub}(\lambda, j)$ durch die Formel

$$\varphi_{hub}(\lambda, j) = \left( (-\varphi_{ist}(\lambda, j)) + \varphi_{zus}(\lambda, j) \right) \bmod (2\pi)$$

bestimmt werden.

In weiteren Ausführungsformen des Verfahrens zur Kompensation von Phasenstörung und/oder zur Implementation mindestens einer optischen Funktion, in denen es nur gewünscht ist, die Phasenstörung $\varphi_{ist}$ für eine Wellenlänge $\lambda$ und einer optischen Faser $j$ zu kompensieren, ohne eine zusätzliche optische Funktion zu implementieren, kann die zusätzliche optische Funktion $\varphi_{zus}(\lambda, j) = 0$ gesetzt werden, wodurch der gewünschte Phasenhub durch die Formel $\varphi_{hub}(\lambda, j) = (-\varphi_{ist}(\lambda, j)) \bmod (2\pi)$ beschreibbar ist, und die Phasenstörung $\varphi_{ist}$ vollständig kompensiert wird.

Da die Stellgröße $x_j$ im Allgemeinen nicht für jede Wellenlänge einzeln variiert werden kann, ist es im Allgemeinen nicht möglich, für jede Wellenlänge und für jede optische Faser der fünften Teilmenge den idealen Zustand $\varphi_{soll} = \varphi_{res}$ zu erreichen, weshalb es notwendig ist, die Fehlerfunktion $f$ zu minimieren, um dem idealen Zustand so nah wie möglich zu kommen.

In bevorzugten Ausführungsformen des Verfahrens zur Kompensation von Phasenstörung und/oder zur Implementation mindestens einer optischen Funktion wird die Fehlerfunktion $f$

- durch das Ziehen einer Quadratwurzel von über alle der Wellenlängen aufsummierten Quadraten der Abweichung zwischen der resultierenden Phase $\varphi_{res}$ und der gewünschten modulierten Phase $\varphi_{soll}$ oder
- durch das Aufsummieren über alle der Wellenlängen, der Beträge der Abweichung zwischen der resultierenden

Phase $\varphi_{res}$ und der gewünschten modulierten Phase $\varphi_{soll}$,

für jeder der optischen Fasern der fünften Teilmenge ermittelt.

Die Fehlerfunktion $f$ kann mittels der Formel

$$f = \sqrt{\sum_{i=1}^{i \geq 2} \left( (\varphi_{res_i} - \varphi_{soll_i}) \bmod (2\pi) \right)^2}$$

oder mittels der Formel

$$f = \sum_{i=1}^{i \geq 2} \left| (\varphi_{res_i} - \varphi_{soll_i}) \bmod (2\pi) \right|$$

ermittelt werden. Dabei stellt $i$ einen Index einer der Wellenlängen dar, $\varphi_{solli}$ und $\varphi_{resi}$ jeweils die gewünschte modulierten Phase und die resultierende Phase für die Wellenlänge mit Index i und der Ausdruck $i \geq 2$ repräsentiert die Anzahl der mindestens zwei Wellenlängen.

In bevorzugten Ausführungsformen des Verfahrens zur Kompensation von Phasenstörung und/oder zur Implementation mindestens einer optischen Funktion wird der resultierende Wert der Stellgröße $x_{j\_fmin}$ für jede der optischen Fasern der fünften Teilmenge durch ein iteratives Verfahren ermittelt, wobei durch das iterative Verfahren die Fehlerfunktion $f$ minimiert wird. Ein Vorteil iterativer Verfahren gegenüber rechnerischen Verfahren ist, dass erstere gegenüber Modellfehlern robust sind, während die Präzision rechnerischer Verfahren durch die Akkuratesse der mathematischen Modelle begrenzt ist, auf denen sie basieren.

In bevorzugten Ausführungsformen des Verfahrens zur Kompensation von Phasenstörung und/oder zur Implementation mindestens einer optischen Funktion wird der resultierende Wert der Stellgröße $x_{j\_fmin}$ für jeden der optischen Fasern der fünften Teilmenge durch ein iteratives Verfahren, umfassend die Schritte

    a) Messen in einer Ebene hinter dem ersten Ende oder hinter dem zweiten Ende des Bildwellenleiters oder des das Element aufweisenden Bildwellenleiters, von Intensität durch jedem der optischen Fasern der vierten Teilmenge geleiteter elektromagnetischer Strahlung in jeder der Wellenlängen,
    b) Ermittlung der Differenz zwischen der gemessenen Intensität und der mit der gewünschten modulierten Phase $\varphi_{soll}$ erwarteten Intensität, für jede der Wellenlängen und jedem der optischen Fasern der vierten Teilmenge,
    c) Ändern des Wertes der Stellgröße $x_j$ für jeder der optischen Fasern der fünften Teilmenge,
    d) Ausführen der Schritte a), b) und c), bis ein lokales Minimum oder das globale Minimum der Differenz zwischen der gemessenen Intensität und der mit der gewünschten modulierten Phase $\varphi_{soll}$ erwarteten Intensität ermittelt ist und Einstellen der Stellgröße $x_j$ auf den Wert, bei welchem das ermittelte lokale Minimum oder globale Minimum erreicht wird, für jeden der optischen Fasern der fünften Teilmenge

erm ittelt.

In bevorzugten Ausführungsformen des Verfahrens zur Kompensation von Phasenstörung und/oder zur Implementation mindestens einer optischen Funktion weist die Stellgröße $x_j$ einen funktionalen Zusammenhang mit

    a) einer Weglängendifferenz $\Delta S_j$ und/oder
    b) einer elektrischen Spannung $U_j$ und/oder
    c) einer elektrischen Stromstärke $I_j$ und/oder
    d) einer Strompulsweite $P_{Ij}$ und/oder
    e) einer Spannungspulsweite $P_{Uj}$ und/oder
    f) einer Temperatur $T_j$ und/oder
    g) einen SLM-Graustufenwert

auf.

Die Stellgröße $x_j$ kann durch unterschiedliche Methoden eingestellt werden.

In Ausführungsformen, falls sie mittels einer Verkürzung und/oder Verlängerung der optischen Fasern der fünften Teilmenge eingestellt wird, weist die Stellgröße $x_j$ einen funktionalen Zusammenhang mit einer durch die Verkürzung und/oder Verlängerung verursachten Weglängendifferenz $\Delta S$; auf. Auch weist die Stellgröße $x_j$ einen funktionalen Zusammenhang mit einer Weglängendifferenz $\Delta S$; auf, wenn sie durch additive, oder ablative Fertigung eines transmissiven Elements eingestellt wird.

In weiteren Ausführungsformen, falls die Stellgröße $x_j$ mittels eines Flächenlichtmodulators eingestellt wird, kann sie einen funktionalen Zusammenhang mit einer oder mehreren physikalischen Größe oder Größen aufweisen, mit der oder denen der Flächenlichtmodulator angesteuert wird. Diese Größe oder Größen kann oder können eine elektrischen Spannung $U_j$ und/oder einer elektrischen Stromstärke $I_j$ und/oder eine Strompulsweite $P_{Ij}$ und/oder eine Spannungspulsweite $P_{Uj}$ und/oder eine Temperatur $T_j$ sein. Dabei kann diese Größe oder können diese Größen auch einen funktionalen Zusammenhang mit einer Weglängendifferenz $\Delta S_j$ aufweisen. Dies ist beispielsweise der Fall, wenn ein Flächenlichtmodulator eine Anordnung separat ansteuerbarer, absenkbarer, anhebbarer und/oder kippbarer Mikrospiegel aufweist.

Die Stellgröße $x_j$ kann in Ausführungsformen mit Flächenlichtmodulatoren durch Strom- oder Spannungspulsweitenmodulation gesteuert werden.

Die Stellgröße $x_j$ kann in weiteren Ausführungsformen mit Flächenlichtmodulatoren durch Temperaturmodulation gesteuert werden, wobei die Temperatur in einem funktionalen Zusammenhang mit einer Stromstärke und/oder einer Spannung steht. Thermo-optisch modulierte Flächenlichtmodulatoren beispielsweise können durch die Temperatur geregelt werden, die Temperatur kann wiederum durch eine elektrische Stromstärke geregelt werden.

In Ausführungsformen kann jedes Element eines Flächenlichtmodulators Graustufenwerte im Bereich 0 bis 255 einnehmen.

Die Phasenänderung $\varphi_{stell}$ einer optischen Faser mit Index $j$ bei einer Wellenlänge $\lambda$, kann durch die Formel

$$\varphi_{stell}(\lambda, j) \;=\; \frac{x_j(n(\lambda,j)-n_U(\lambda))}{\lambda} \bmod (2\pi)$$

beschrieben werden, wobei $n(\lambda,j)$ der Brechungsindex des optischen Faser $j$ bei der Wellenlänge $\lambda$ für das Materials ist, auf das die Stellgröße $x_j$ angelegt wird und $n_U(\lambda)$ der Brechungsindex des den Bildwellenleiter umgebenden Mediums bei der Wellenlänge $\lambda$.

Andere Beschreibungen der Phasenänderung $\varphi_{stell}$ sind nicht ausgeschlossen.

Im Allgemeinen können verschiedene Werte der Stellgröße $x_j$ dieselbe Phasenänderung $\varphi_{stell}$ für eine Wellenlänge zum Ergebnis haben. Dieses Prinzip macht sich das Verfahren zur Kompensation von Phasenstörung und/oder zur Implementation mindestens einer optischen Funktion zu Nutze, um einen Wert der Stellgröße $x_j$ zu ermitteln, bei der die resultierende Phase $\varphi_{res} = (\varphi_{ist} + \varphi_{stell})\bmod(2\pi)$ der gewünschten modulierten Phase $\varphi_{soll}$ für alle der Wellenlängen möglichst nahe kommt. Überraschenderweise ist dies der bei Werten der Stellgröße $x_j$ der Fall, bei denen die Phasenänderung $\varphi_{stell}$ weit über $2\pi$ liegen würde, wenn sie nicht den Modulo-Operator $\bmod(2\pi)$ umfassen würde.

In bevorzugten Ausführungsformen des Verfahrens zur Kompensation von Phasenstörung und/oder zur Implementation mindestens einer optischen Funktion werden die funktionalen Zusammenhänge zwischen der Stellgröße $x_j$ und den in a) bis f) genannten Größen jeweils durch eine Kalibration ermittelt und/oder umfasst der funktionale Zusammenhang zwischen der Stellgröße $x_j$ und der Weglängenänderung $\Delta S_j$ die auf die jeweilige Wellenlänge normierte Differenz zwischen

    a) dem Brechungsindex der verlängerten und/oder verkürzten optischen Fasern und/oder des Elements und

    b) dem Brechungsindex des den Bildwellenleiter umgebenden Mediums.

Falls die Stellgröße $x_j$ durch einen eine Mikrospiegelanordnung umfassenden Flächenlichtmodulator eingestellt wird, kann die Phasenänderung $\varphi_{stell}$ beispielsweise durch $\varphi_{stell}(\lambda, j) \;=\; 2\pi \left( \dfrac{x_j}{\lambda} \bmod 1 \right)$ beschrieben werden, wobei die Stellgröße $x_j$ mit der Weglängendifferenz $\Delta S_j$ den funktionalen Zusammenhang $x_j = n_U(\lambda)\,\Delta S_j$ aufweist, $\dfrac{x_j}{\lambda}$ kann dabei Werte zwischen einschließlich -9 und einschließlich +9 einnehmen.

Falls die Stellgröße $x_j$ durch eine Verkürzung und/oder Verlängerung der optischen Fasern der fünften Teilmenge und/oder durch ein transmissives Element eingestellt wird, kann die Phasenänderung $\varphi_{stell}$ beispielsweise durch

$$\varphi_{stell}(\lambda, j) \;=\; 2\pi \left( \frac{x_j}{\lambda} \bmod 1 \right)$$

beschrieben werden, wobei die Stellgröße $x_j$ mit der Weglängendifferenz $\Delta S_j$ den funktionalen Zusammenhang $x_j = ((n(\lambda, j) - n_U(\lambda))\Delta S_j$ aufweist, $\dfrac{x_j}{\lambda}$ kann dabei Werte zwischen einschließlich -9 und einschließlich +9 einnehmen.

In Ausführungsformen des Verfahrens erfolgt die Kompensation von Phasenstörung und/oder die Implementation einer optischen Funktion durch ein statisches Element, welches entweder ein transmissives oder ein reflektives Element ist und/oder durch ein adaptives Element, welches entweder ein transmissives oder ein reflektives Element ist, wobei ein reflektives Element von dem jeweiligen Ende des Bildwellenleiters, hinter welchem es positioniert ist, beabstandet positioniert ist und durch Reflexion elektromagnetischer Strahlung der mindestens zwei Wellenlängen aus einem geeigneten Einfallswinkel, eine Phasenmaske auf das jeweilige Ende des Bildwellenleiters abbildet.

Zweckmäßig ist ein adaptives Element als Flächenlichtmodulator ausgebildet, wobei der Flächenlichtmodulator ein elektro-optisch modulierter Flächenlichtmodulator, oder ein thermo-optisch modulierter Flächenlichtmodulator ist. Vorteilhaft ist der Flächenlichtmodulator als LCoS ausgebildet.

Der Abstand des reflektiven Elements von dem jeweiligen Ende des Bildwellenleiters, hinter welchem es positioniert ist, kann nach Belieben gewählt werden. Bevorzugt liegt der Abstand innerhalb des Bereichs zwischen dem 10.000-fachen der kleinsten der Wellenlängen und dem 10.000.000-fachen der größten der Wellenlängen, besonders bevorzugt innerhalb des Bereichs zwischen dem 10.000-fachen der kleinsten der Wellenlängen und dem 100.000-fachen der größten der Wellenlängen.

Der geeignete Einfallswinkel elektromagnetischer Strahlung auf das reflektive Element liegt über 0° und unter 90°, vorzugsweise zwischen 10° und 80°.

Bevorzugt erfolgt

- die Verlängerung der optischen Fasern der fünften Teilmenge zur Kompensation von Phasenstörung und/oder zur Implementation mindestens einer optischen Funktion durch eine additive Fertigung auf die optischen Fasern der fünften Teilmenge am ersten Ende und/oder am zweiten Ende des Bildwellenleiters und/oder
- die Verkürzung der optischen Fasern der fünften Teilmenge zur Kompensation der Phasenstörung und/oder zur Implementation einer optischen Funktion durch Laser-Ablation und/oder durch Elektronenstrahlablation der optischen Fasern der fünften Teilmenge am ersten Ende und/oder dem zweiten Ende des Bildwellenleiters und/oder
- das Bereitstellen des Elements am ersten Ende und/oder am zweiten Ende des Bildwellenleiters durch eine additive Fertigung auf einem Element-Rohling und/oder durch Laser-Ablation und/oder durch Elektronenstrahlablation eines Element-Rohlings und/oder
- das Bereitstellen des Elements an dem ersten Ende und/oder dem zweiten Ende des Bildwellenleiters durch Fertigung von Metaoptiken, wobei die Metaoptiken dadurch gekennzeichnet sind, dass sie Strukturen aufweisen, deren Dimensionen kleiner als die kleinste der Wellenlängen sind.

Als Element-Rohling wird im Sinne des Verfahrens zur Kompensation von Phasenstörung von mindestens zwei Wellenlängen $\lambda_k$ des zumindest einen Bildwellenleiters und/oder zur Implementation mindestens einer optischen Funktion das Element in dem Zustand bezeichnet, in dem es sich zeitlich vor der additiven Fertigung und/oder der Laser-Ablation und/oder der Elektronenstrahlablation befindet, durch welche es zu einem Element zur Kompensation von Phasenstörung von mindestens zwei Wellenlängen eines Bildwellenleiters und/oder zur Implementation mindestens einer optischen Funktion gefertigt wird.

In bevorzugten Ausführungsformen des Verfahrens umfasst die additive Fertigung Ein-Photon-Polymerisation und/oder Zwei-Photonen-Polymerisation und/oder Mehr-Photonen-Polymerisation.

In bevorzugten Ausführungsformen des Verfahrens zur Kompensation von Phasenstörung von mindestens zwei Wellenlängen $\lambda_k$ des zumindest einen Bildwellenleiters und/oder zur Implementation mindestens einer optischen Funktion erfolgt Teilschritt b) i) des erfindungsgemäßen Verfahrens entweder mittels Weißlichtinterferometrie oder mittels digitaler Holographie und/oder eines Phase-Retrieval-Verfahrens.

Digitale Holographie und das Phase-Retrieval-Verfahrens können dabei gemeinsam verwendet werden.

Ein weiterer Aspekt der Erfindung betrifft eine Vorrichtung zur Kompensation von Laufzeitunterschieden und/oder zur Implementation eines gewünschten Laufzeitprofils zumindest eines wenigstens zwei optische Fasern aufweisenden Bildwellenleiters, umfassend eine zum Vermessen des Laufzeitunterschiedes von Bildwellenleitern in mindestens einer Wellenlänge geeignete Anordnung, wobei

- die zum Vermessen des Laufzeitunterschiedes des Bildwellenleiters geeignete Anordnung eine zur Änderung der effektiven Brechungsindizes optischer Fasern geeignete Quelle hochenergetischer elektromagnetischer Strahlung umfasst und die Quelle

    ◦ als Strahlungsquelle für das Vermessen des Laufzeitunterschiedes der optischen Fasern und einer gleichzeitigen Änderung des effektiven Brechungsindizes der optischen Fasern verwendbar ist und/oder
    ◦ durch Herunterregeln der Strahlungsleistung und/oder durch die Implementation eines optischen Filters zwischen der Quelle und dem Bildwellenleiter als Quelle niederenergetischer Strahlung betreibbar und für das Vermessen des Laufzeitunterschiedes der optischen Fasern verwendbar ist und/oder

- die Vorrichtung eine von der Anordnung separate zur Änderung der effektiven Brechungsindizes optischer Fasern geeignete Quelle hochenergetischer Strahlung umfasst,

ferner umfassend mindestens eine erste Positionierungsvorrichtung, die dazu geeignet ist

- den Bildwellenleiter und die Anordnung so zueinander zu positionieren, um das Vermessen der Laufzeitunterschiedes und/oder die Änderung der effektiven Brechungsindizes von optischen Fasern des Bildwellenleiters zu ermöglichen und/oder
- den Bildwellenleiter und die Quelle hochenergetischer elektromagnetischer Strahlung so zueinander zu positionieren, dass die longitudinale Einkopplung von durch die Quelle emittierbarer Strahlung in mindestens eine optische Faser ermöglicht ist.

Die Laufzeitunterschiede der optischen Fasern des Bildwellenleiters zur Referenzlaufzeit sind in Ausführungsformen der Vorrichtung einzeln vermessbar.

In Ausführungsformen der Vorrichtung ist die hochenergetische Strahlung einzeln in die Fasern des Bildwellenleiters einkoppelbar und dabei jeweils mit unterschiedlichen Eigenschaften beaufschlagbar, die jeweils zu einer Annäherung des effektiven Brechungsindizes der jeweiligen optischen Faser an den effektiven Brechungsindex erlauben, der für die jeweilige gewünschte Laufzeit benötigt wird.

Die Quelle hochenergetischer elektromagnetischer Strahlung umfasst in Ausführungsformen mindestens einen ultra-kurz gepulsten Laser, und/oder mindestens eine UV-Lichtquelle, insbesondere mindestens einen Femtosekundenlaser und/oder mindestens eine Excimer-Lichtquelle, wobei die Excimer-Lichtquelle bevorzugt eine 146-nm-Excimer-Lichtquelle oder eine 248-nm-Excimer-Lichtquelle ist und wobei die Excimer-Lichtquelle mindestens eine Excimerlampe und/oder mindestens einen Excimerlaser umfasst.

Der ultra-kurz gepulsten Laser ermöglicht in Ausführungsformen die Emission von Pulsen mit einer Pulsdauer zwischen 10 fs und 10 ps und der Femtosekundenlaser ermöglicht die Emission von Pulsen mit einer Pulsdauer zwischen 10 fs bis 1 ps.

In Ausführungsformen der Vorrichtung ist die Quelle hochenergetischer elektromagnetischer Strahlung zu einer Modulation einer oder mehrerer Stellgrößen der Strahlung, ausgewählt aus der der Leistung, der Energie, der Pulsdauer, der Pulsform, dem spektralen Bereich, dem spektralen Verlauf der Leistung, dem zeitlichen Verlauf der Leistung, dem spektralen Verlauf der Energie, dem zeitlichen Verlauf der Energie und der Polarisation ausgebildet.

Zweckmäßig ist eine kontinuierlich emittierenden UV-Excimerlampe beispielsweise dazu ausgebildet

- mit geringer Leistung über einen langen Zeitraum hinweg, oder
- mit hoher Leistung über einen kurzen Zeitraum hinweg

UV-Excimer-Licht zu emittieren und in eine optische Faser einzukoppeln, um den effektiven Brechungsindex der optischen Faser zu ändern. Es ist beispielsweise auch möglich, dass ein gepulster IR-Laser oder ein gepulster sichtbarer Laser mit einer Pulsleistung von 10 MW dazu ausgebildet ist, gepulstes IR-Laserlicht zu emittieren und in eine optische Faser einzukoppeln, um den effektiven Brechungsindex der optischen Faser zu ändern.

Die Vorrichtung weist in Ausführungsformen mindestens eine $H_2$- und/oder $N_2$-Kammer auf, wobei die $H_2$- und/oder $N_2$-Kammer einen luftdicht verschließbaren Gasbehälter und eine an den Gasbehälter anschließbare, zum Leiten von $H_2$- und/oder $N_2$-Gas geeignete Leitung umfasst, wobei die Leitung an ein $H_2$- und/oder $N_2$-Gasnetz anschließbar ist und/oder an einem zum Beinhalten von $H_2$- und/oder $N_2$-Gas geeigneten Druckbehälter anschließbar ist und bevorzugt ein zum Herausbefördern des im Gasbehälter befindlichen Gases geeignetes Gerät und/oder ein zum Hereinbefördern des $H_2$- und/oder $N_2$-Gases geeignetes Gerät umfasst und wobei die Kammer dazu ausgebildet ist, den zumindest einen Bildwellenleiter zu beinhalten und die Kammer vorteilhaft mindestens einen für zumindest die Halbwertsbreite

- der zum Vermessen des Laufzeitunterschiedes verwendbare Strahlung und
- der hochenergetischen Strahlung

transparenten Bereich aufweist und die erste Positionierungsvorrichtung in der Kammer angeordnet ist oder die Kammer mindestens eine zweite Positionierungsvorrichtung aufweist, die dazu ausgebildet ist, den Bildwellenleiter so innerhalb der Kammer zu positionieren, dass die hochenergetische Strahlung und die zum Vermessen des Laufzeitunterschiedes verwendbare Strahlung in den Bildwellenleiter longitudinal einkoppelbar ist.

In Ausführungsformen der Vorrichtung ist die Modulation des zeitlichen und spektralen Verlaufs der Strahlungsleistung des ultra-kurz gepulsten Lasers vorteilhaft so ausbildbar, dass die über das gesamte Spektrum integrierte Strahlungsleistung bei longitudinaler Einkopplung der Strahlung in die mindestens eine optische Faser des Bildwellenleiters an einem auswählbaren Abstand vom ersten Ende des Bildwellenleiters innerhalb der mindestens einen optischen Faser einen Maximalwert annimmt.

Die Vorrichtung weist in Ausführungsformen

- mindestens eine Apparatur auf, welche dazu ausgebildet ist, die Kerne der optischen Fasern des Bildwellenleiters am ersten Ende und/oder am zweiten Ende aufzuweiten und/oder
- mindestens einen für das Beinhalten einer Immersionsflüssigkeit, insbesondere eines Immersionsöls geeigneten Flüssigkeitsbehälter auf, der dazu ausgebildet ist, zumindest das erste Ende und/oder zumindest das zweite Ende des zumindest einen Bildwellenleiters zu beinhalten und mindestens einen für zumindest die Halbwertsbreite des Spektralbereichs

  ∘ der zum Vermessen der Laufzeitunterschiede verwendbare Strahlung und
  ∘ der hochenergetischen Strahlung

  transparenten Bereich aufweist und die erste Positionierungsvorrichtung im Flüssigkeitsbehälter angeordnet ist oder der Flüssigkeitsbehälter mindestens eine dritte Positionierungsvorrichtung aufweist, die dazu ausgebildet ist, zumindest das erste Ende und/oder zumindest das zweite Ende des Bildwellenleiters so innerhalb der Kammer zu positionieren, dass die zum Vermessen des Laufzeitunterschiedes verwendbare Strahlung und die hochenergetischen Strahlung in den Bildwellenleiter longitudinal einkoppelbar ist und/oder

- mindestens eine Glasplatte auf, wobei die Glasplatte und/oder der Bildwellenleiter so positionierbar sind/ist, dass die Glasplatte in Berührungskontakt mit dem ersten Ende und/oder dem zweiten Ende des Bildwellenleiters steht,

wobei die Materialien, aus dem die Immersionsflüssigkeit und/oder die Glasplatte bestehen, mindestens jeweils ein Material umfassen, dessen Brechungsindex dem effektiven Brechungsindex mindestens einer optischen Faser des Bildwellenleiters beliebig nah ist und das jeweilige Material zumindest für die Halbwertsbreite der Wellenlänge der elektromagnetischen Strahlung transparent ist, die von der zum Vermessen des Laufzeitunterschiedes von Bildwellenleitern geeigneten Anordnung emittierbar und absorbierbar ist.

Die Apparatur, welche dazu ausgebildet ist, die Kerne der optischen Fasern des Bildwellenleiters am ersten Ende und/oder am zweiten Ende aufzuweiten kann ein $CO_2$- Laser sein, der dazu ausgelegt ist, das erste Ende und/oder das zweite Ende des Bildwellenleiters zu erhitzen.

In Ausführungsformen der Vorrichtung umfasst die zum Vermessen des Laufzeitunterschiedes von Bildwellenleitern in mindestens einer Wellenlänge geeignete Anordnung mindestens ein Weißlichtinterferometer und/oder mindestens einen optischen Kohärenztomografen.

In weiteren Ausführungsformen ist die Mehrwellenlängenholographie als off-axis-Holographie unter Verwendung eines Mach-Zehnder-Interferometers ausgebildet.

In Ausführungsformen weist die Vorrichtung mindestens eine erste Quelle elektromagnetischer Strahlung, mindestens einen Y-Wellenleiter, mindestens ein erstes Vergrößerungsobjektiv, mindestens einen Strahlteiler, mindestens einen Spiegel und mindestens einen bildgebenden Detektor für elektromagnetische Strahlung auf. Falls die erste Quelle elektromagnetischer Strahlung nicht als Quelle hochenergetischer elektromagnetischer Strahlung ausbildbar ist, weist die Vorrichtung ferner eine zweite Quelle elektromagnetischer Strahlung auf, die als Quelle hochenergetischer elektromagnetischer Strahlung ausbildbar ist Die erste Quelle elektromagnetischer Strahlung und der Y-Wellenleiter sind so zueinander positionierbar, dass elektromagnetische Strahlung von der ersten Quelle in ein erstes Ende des Y-Wellenleiters einkoppelbar und aufteilbar ist, sowie an einem zweiten Ende und einem dritten Ende auskoppelbar ist. Das zweite Ende des Y-Wellenleiters und das erste Vergrößerungsobjektiv sind so ausgebildet, dass ein bereitgestellter Bildwellenleiter so zum zweiten Ende des Y-Wellenleiters und zum ersten Vergrößerungsobjektiv positioniert werden kann, dass elektromagnetische Strahlung vom zweiten Ende des Y-Wellenleiters in ein erstes Ende des Bildwellenleiters einkoppelbar ist und von einem zweiten Ende des Bildwellenleiters in das erste Vergrößerungsobjektiv einkoppelbar ist. Das erste Vergrößerungsobjektiv und der Strahlteiler sind so zueinander anordenbar, dass aus dem ersten Vergrößerungsobjektiv austretende elektromagnetische Strahlung auf den Strahlteiler treffen kann, wobei der Strahlteiler dazu ausgebildet ist, $X$ % der auf ihn treffenden elektromagnetischen Strahlung zu transmittieren und $(100 - X)$ % zu reflektieren. Der Strahlteiler und der bildgebende Detektor sind so zueinander positionierbar, dass aus dem ersten Vergrößerungsobjektiv austretende, durch den Strahlteiler transmittierbare elektromagnetische Strahlung auf den bildgebenden Detektor treffen kann. Das Dritte Ende des Y-Wellenleiters, der Strahlteiler und der Spiegel sind so zueinander anordenbar, dass aus dem dritten Ende des Y-Wellenleiters auskoppelbare elektromagnetische Strahlung zu $X$ % vom Strahlteiler transmittierbar und durch den Spiegel zum Strahlteiler reflektierbar ist. Der Strahlteiler und der bildgebende Detektor sind so anordenbar, dass die vom Spiegel reflektierbare Strahlung zu $(100 - X)$ % durch den Strahlteiler so reflektierbar ist, dass sie auf den bildgebenden Detektor trifft. Der Spiegel ist entlang der optischen Achse des aus dem dritten Ende des Y-Wellenleiters auskoppelbaren und durch den Strahlteiler transmittierbaren Lichtes beweglich positionierbar, so dass die Laufzeit der aus dem dritten Ende des Y-Wellenleiters austretenden und auf den bildgebenden Detektor gelangende elektromagnetische Strahlung bezüglich zur Laufzeit der aus dem zweiten Ende des Y-Wellenleiters austretenden und auf den bildgebenden Detektor gelangenden elektromagnetische Strahlung veränderbar ist. Das erste Vergrößerungsobjektiv und der bildgebende Detektor sind dafür ausgebildet und so zueinander und zum Bildwellenleiter anordenbar, dass an Strukturen, wie Interferenzmuster an den Facetten der optischen Fasern vom bildgebenden Detektor auflösbar sind.

X kann eine beliebige reelle Zahl im Bereich $0 < X < 100$ sein. Bevorzugt betragen der erste Teil und der zweite Teil des Lichtes jeweils 50% des in den Y-Wellenleiter eingekoppelten Teil des Lichtes. Bevorzugt ist $X = 50$. Bevorzugt umfasst das Licht von einer Superlumineszenzdiode emittiertes Licht. Weitere optische Komponenten im Strahlengang, wie Polarisationsfilter, Linsen, Strahlteiler und/oder Spiegel werden in Ausführungsformen der Vorrichtung nicht ausgeschlossen.

Hochenergetische elektromagnetische Strahlung ist durch das erste Vergrößerungsobjektiv in einzelne optische Fasern des Bildwellenleiters einkoppelbar.

Die Quelle hochenergetischer elektromagnetischer Strahlung, das erste Vergrößerungsobjektiv sind in Ausführungsformen ausgebildet und so zueinander und zum Bildwellenleiter positionierbar, dass die von der ersten oder zweiten Quelle emittierte hochenergetische Strahlung in einzelne optische Fasern des Bildwellenleiters einkoppelbar ist und an einzelnen optischen Fasern des Bildwellenleiters. Die relative Position der Quelle hochenergetischer elektromagnetischer Strahlung, des Bildwellenleiters und des ersten Vergrößerungsobjektivs zueinander und zum Bildwellenleiter ist so veränderbar, dass die hochenergetische elektromagnetische Strahlung in weitere optische Fasern des Bildwellenleiters einkoppelbar ist.

In Ausführungsformen ist ein Flächenlichtmodulator ist so im Strahlengang der hochenergetischen elektromagnetischen Strahlung angeordnet und so einstellbar, dass die hochenergetische Strahlung der Quelle in jeweils eine einzelne optische Faser des Bildwellenleiters einkoppelbar ist. Die Einstellung des Flächenlichtmodulators dabei so veränderbar, dass die hochenergetische elektromagnetische Strahlung in weitere optische Fasern des Bildwellenleiters einkoppelbar ist.

In Ausführungsformen weist die Vorrichtung eine Einrichtung zur Kompensation von elektromagnetischer Phasenstörung von mindestens zwei Wellenlängen $\lambda_k$ des zumindest einen Bildwellenleiters und/oder zur Implementation einer Funktion, welche Ausbreitungsrichtungen elektromagnetischer Strahlung von mindestens einer Wellenlänge $\lambda_f$ beim Ein- und/oder Austritt in den und/oder aus dem Bildwellenleiter ändert, umfassend eine zum Vermessen der Phasenstörung von Bildwellenleitern in mindestens zwei Wellenlängen geeignete Anordnung, ferner umfassend

- ein Element, welches zur Kompensation von elektromagnetischer Phasenstörung von mindestens zwei Wellenlängen $\lambda_k$ und/oder zur Implementation einer Funktion, welche Ausbreitungsrichtungen elektromagnetischer Strahlung von mindestens einer Wellenlänge $\lambda_f$ beim Ein- und/oder Austritt in den und/oder aus dem Bildwellenleiter ändert geeignet ist, wobei das Element an einem ersten Ende und/oder einem zweiten Ende des Bildwellenleiters positionierbar ist und derart moduliert oder modulierbar ist, dass das Element entlang der elektromagnetischen Ausbreitungsrichtung eines oder mehrerer ausgewählter Wellenleiter eine Stellgröße $x_{j\_fmin}$ aufweist und/oder
- ein zur Verkürzung und/oder Verlängerung optischer Fasern von Bildwellenleitern geeignetes Gerät, wobei das Gerät, wobei der Bildwellenleiter und das Gerät so zueinander positionierbar sind, dass eine Verkürzung und/oder Verlängerung von optischen Fasern des Bildwellenleiters möglich, so, dass einer Verkürzung und/oder Verlängerung unterzogene optische Fasern eine Stellgröße $x_{j\_fmin}$ aufweisen,

wobei die Stellgröße $x_{j\_fmin}$ mittels Durchführung der Teilschritte a) bis f) des Verfahrens zur Kompensation von Phasenstörung von mindestens zwei Wellenlängen $\lambda_k$ des zumindest einen Bildwellenleiters und/oder zur Implementation mindestens einer optischen Funktion, welche Ausbreitungsrichtungen elektromagnetischer Strahlung von mindestens einer Wellenlänge $\lambda_f$ beim Ein- und/oder Austritt in den und/oder aus dem Bildwellenleiter ändert.

In Ausführungsformen der Einrichtung ist die gewünschte modulierte Phase $\varphi_{soll}$ durch die Formel $\varphi_{soll}(\lambda, j) = (\varphi_{ist}(\lambda, j) + \varphi_{hub}(\lambda, j)) \bmod (2\pi)$ beschreibbar, wobei $\varphi_{hub}(\lambda, j)$ ein gewünschter Phasenhub ist.

Eine Änderung der elektromagnetischen Weglänge, welche in der gewünschten modulierten Phase $\varphi_{soll}$ resultiert, kann durch die Formel

$$L_{soll}(\lambda, j) = \frac{\lambda \, \varphi_{hu}(\lambda, j)}{2\pi} + N\,\lambda$$

beschrieben werden, wobei $N$ eine beliebige ganze Zahl ist.

In weiteren Ausführungsformen der Einrichtung liegt $N$ in dem Bereich zwischen einschließlich -9 und einschließlich +9.

In Ausführungsformen der Einrichtung, die dazu ausgelegt sind, die Phasenstörung $\varphi_{ist}$ für eine Wellenlänge $\lambda$

und einen Wellenleiter $j$ zu kompensieren und auch eine zusätzliche Funktion $\varphi_{zus}$ zu implementieren, kann der gewünschte Phasenhub $\varphi_{hu}(\lambda, j)$ durch die Formel

$$\varphi_{hub}(\lambda, j) = \left(-\varphi_{ist}(\lambda, j) + \varphi_{zus}(\lambda, j)\right) \mathrm{mod}\,(2\pi)$$

beschrieben werden.

In weiteren Ausführungsformen der Einrichtung, die dazu ausgelegt sind, die Phasenstörung $\varphi_{ist}$ für eine Wellenlänge $\lambda$ und einen Wellenleiter $j$ zu kompensieren, ohne eine zusätzliche Funktion zu implementieren, kann die zusätzliche Funktion $\varphi_{zus}(\lambda, j) = 0$ gesetzt sein, wodurch der gewünschte Phasenhub durch die Formel $\varphi_{hub}(\lambda, j) = (-\varphi_{ist}(\lambda, j)) \mathrm{mod}\,(2\pi)$ beschreibbar ist, und die Phasenstörung $\varphi_{ist}$ vollständig kompensiert ist.

In bevorzugten Ausführungsformen der Einrichtung weist die Stellgröße $x_j$ einen funktionalen Zusammenhang mit

a) einer Weglängendifferenz $\Delta S_j$ und/oder
b) einer elektrischen Spannung $U_j$ und/oder
c) einer elektrischen Stromstärke $I_j$ und/oder
d) einer Strompulsweite $P_{Ij}$ und/oder
e) einer Spannungspulsweite $P_{Uj}$ und/oder
f) einer Temperatur $T_j$ und/oder
g) einen SLM-Graustufenwert

auf.

Im Allgemeinen können verschiedene Werte der Stellgröße $x_j$ dieselbe Phasenänderung $\varphi_{stell}$ für eine Wellenlänge zum Ergebnis haben. Dieses Prinzip macht sich die erfindungsgemäße Einrichtung zu Nutze, um einen Wert der Stellgröße $x_j$ zu ermöglichen, bei der die resultierende Phase $\varphi_{res} = (\varphi_{ist} + \varphi_{stell}) \mathrm{mod}(2\pi)$ der gewünschten modulierten Phase $\varphi_{soll}$ für alle der Wellenlängen möglichst nahe ist. Überraschenderweise ist dies der bei Werten der Stellgröße $x_j$ der Fall, bei denen die Phasenänderung $\varphi_{stell}$ weit über $2\pi$ liegen würde, wenn sie nicht den Modulo-Operator $\mathrm{mod}(2\pi)$ umfassen würde.

In bevorzugten Ausführungsformen der Einrichtung, die ein Element umfassen, ist das Element ein statisches Element, welches entweder ein transmissives oder ein reflektives Element ist und/oder ein adaptives Element, welches entweder ein transmissives oder ein reflektives Element ist, wobei ein reflektives Element von dem jeweiligen Ende der Anordnung, hinter welchem es positioniert ist, beabstandet positioniert ist und durch Reflexion elektromagnetischer Strahlung der mindestens zwei Wellenlängen aus einem geeigneten Einfallswinkel, eine Phasenmaske auf das jeweilige Ende der Anordnung abbildet, wobei das adaptive Element als Flächenlichtmodulator ausgebildet ist.

In bevorzugten Ausführungsformen der Einrichtung, die ein Element umfassen, ist das Element ein statisches Element, wobei das Element entlang der elektromagnetischen Ausbreitungsrichtung jedes der ausgewählten Wellenleiter eine Weglängendifferenz $\Delta S_j$ bezüglich zu einer Referenzlänge aufweist. Es kann dabei eine beliebige Referenzlänge gewählt werden. Die Weglängendifferenz $\Delta S_j$ für jeden der ausgewählten Wellenleiter ist durch eine als Phasenmaske ausgebildete Oberflächenbeschaffung des Elements realisiert.

In Ausführungsformen der Einrichtung umfasst das Material des Elements zur Kompensation der Phasenstörung der ausgewählten Wellenleiter am ersten Ende und/oder am zweiten Ende der jeweiligen Wellenleiters Metaoptiken, wobei die Metaoptiken dadurch gekennzeichnet sind, dass sie Strukturen aufweisen, deren Dimensionen kleiner als die kleinste der Wellenlängen sind.

Für Kerne optischer Fasern in Frage kommende Materialien umfassen Siliziumdioxid, Chalkogenide, Fluoridgläser, Fluorozirkonate, Fluoroaluminate, Phosphatgläser, Korunde, Polycarbonat und/oder Polymethylmethacrylat. Andere Materialien sind nicht ausgeschlossen.

In Frage kommende Wellenlängen umfassen den Spektralbereich zwischen 100 nm und 10 $\mu$m, bevorzugt zwischen 140 nm und 3 $\mu$m. Andere Spektralbereiche sind nicht ausgeschlossen.

Ein weiterer Aspekt der Erfindung betrifft eine Verwendung

• des erfindungsgemäßen Verfahrens und/oder seinen Ausführungsformen und/oder
• der erfindungsgemäßen Vorrichtung und/oder seinen Ausführungsformen

zur Kompensation von Laufzeitunterschieden und/oder zur Implementation eines gewünschten Laufzeitprofils eines mindestens zwei optische Fasern aufweisenden Bildwellenleiters in der Krebsdiagnostik, der nichtlinearen Endomikroskopie, der OCT, der Swept-Source-OCT, zur ungestörten Übertragung von Femtosekunden-Pulsen und/oder zur Korrektur von Laufzeitunterschieden, die in Bildwellenleitern auftreten, welche miteinander verdrillte optische Fasern aufweisen.

Die Erfindung ist nicht auf die dargestellten und beschriebenen Ausführungsformen beschränkt, sondern umfasst auch alle im Sinne der Erfindung gleich wirkenden Ausführungsformen. Ferner ist die Erfindung auch nicht auf die speziell beschriebenen Merkmalskombinationen beschränkt, sondern kann auch durch jede beliebige andere Kombination von bestimmten Merkmalen aller insgesamt offenbarten Einzelmerkmale definiert sein, sofern sich die Einzelmerkmale nicht gegenseitig ausschließen, oder eine spezifische Kombination von Einzelmerkmalen nicht explizit ausgeschlossen ist.

**Ausführungsbeispiel**

Nachfolgend soll die Erfindung anhand eines Ausführungsbeispiels eingehender erläutert werden. Das Ausführungsbeispiel bezieht sich auf eine Ausführungsform des erfindungsgemäßen Verfahrens und der erfindungsgemäßen Vorrichtung und soll dabei die Erfindung beschreiben, ohne diese zu beschränken.

Ein als kohärente Bündel optischer Fasern ausgelegter Bildwellenleiter (1, CFB) wird bereitgestellt.

Licht wird durch eine eine Superlumineszenzdiode aufweisende Quelle elektromagnetischer Strahlung (SLED) emittiert und in einen Y-Wellenleiter (Y 50:50) eingekoppelt und aufgeteilt.

Eine erste Hälfte des Lichtes wird aus dem Y-Wellenleiter (Y 50:50) in einen ersten Lichtwellenleiter (SMF1) eingekoppelt und so aus diesem ausgekoppelt, dass es zunächst auf eine erste bikonvexe Linse (L1) trifft und von der ersten bikonvexen Linse (L1) so auf einen ersten Strahlteiler (BS1) trifft, dass der erste Strahlteiler (BS1) 50 % der ersten Hälfte des Lichtes zu einem ersten Spiegel (M1) transmittiert, der erste Spiegel (M1) die 50 % der ersten Hälfte des Lichtes zum ersten Strahlteiler (BS1) reflektiert und der erste Strahlteiler (BS1) 50 % von den 50 % der ersten Hälfte des Lichtes durch einen ersten Polarisationsfilter (PF1) zu einem eine Kamera aufweisenden bildgebenden Detektor für elektromagnetische Strahlung (CAM) reflektiert.

Eine zweite Hälfte des Lichtes wird aus dem Y-Wellenleiter (Y 50:50) in das erste Ende eines am zweiten Ende eine Linse aufweisenden zweiten Lichtwellenleiter (SMF2+Lens) eingekoppelt und am zweiten Ende aus diesem ausgekoppelt. Die zweite Hälfte des Lichtes gelangt anschließend durch einen zweiten Polarisationsfilter (PF2), dann durch einen dritten Polarisationsfilter (PF3) und wird daraufhin in den als kohärentes Bündel optischer Fasern ausgebildeten Bildwellenleiter (1, CFB) gekoppelt. Die zweite Hälfte des Lichtes wird aus dem Bildwellenleiter (1, CFB) ausgekoppelt und gelangt durch ein Mikroskopobjektiv (MO1) und anschließend eine zweite bikonvexe Linse (L2) auf einen zweiten Strahlteiler (BS2), der 50% der zweiten Hälfte des Lichtes zum ersten Strahlteiler (BS1) reflektiert. Der erste Strahlteiler (BS1) transmittiert 50 % der 50% der zweiten Hälfte des Lichtes durchden ersten Polarisationsfilter (PF1) hindurch zu dem bildgebenden Detektor (CAM).

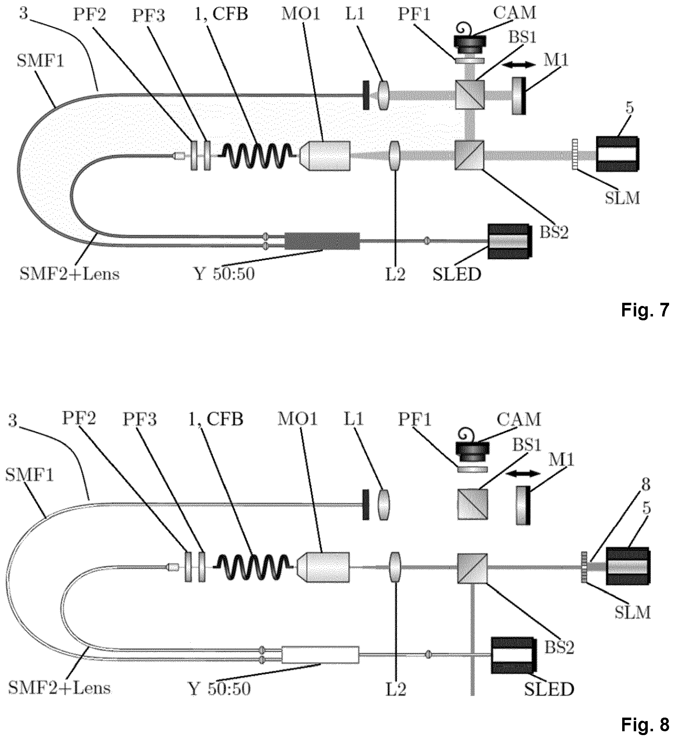

Der Bildwellenleiter (1, CFB), das Mikroskopobjektiv (MO1), die zweite bikonvexe Linse (L2), der zweite Strahlteiler (BS2), der erste Strahlteiler (BS1), der erste Polarisationsfilter (PF1) und der bildgebende Detektor (CAM) sind so zueinander angeordnet und jeweils so ausgewählt, dass durch den bildgebenden Detektor (CAM) Strukturen, wie Interferenzmuster an den Facetten der optischen Fasern aufgelöst werden können. Der erste Spiegel (M1) wird entlang der optischen Achse der 50 % der ersten Hälfte des Lichtes bewegt, bis eine der optischen Fasern aus der Perspektive des bildgebenden Detektors (CAM) ein Interferenzmuster aufweist. Die Laufzeit des Anteils des Lichtes, welches durch die das Interferenzmuster aufweisende optische Faser geleitet wird, kann als Referenzlaufzeit ausgewählt werden. Der erste Spiegel (M1) wird weiter entlang der optischen Achse der 50 % der ersten Hälfte des Lichtes bewegt, bis jede optische Faser des Bildwellenleiters (1, CFB), von der eine Messung des Laufzeitunterschiedes bezüglich zur Referenzlaufzeit erwünscht ist, im Laufe der Bewegung des ersten Spiegels (M1) ein Interferenzmuster aufgewiesen hat. Dabei wird bei jedem Erscheinen eines Interferenzmusters die Position des ersten Spiegels (M1) und der das Interferenzmuster aufweisenden optischen Faser auf einem Speichermedium registriert. Anhand der relativen Position des ersten Spiegels (M1) und der Kenntnis der Lichtgeschwindigkeit in Luft, wird der Laufzeitunterschied bezüglich zur Referenzlaufzeit für jede der optischen Fasern ermittelt, welche bei einer bestimmten Position des ersten Spiegels (M1) ein Interferenzmuster aufweisen. Diese Konfiguration der Vorrichtung (3) wird in Fig. 7 schematsich dargestellt.

Von einer als 248-nm-Excimerlampe ausgebildete Quelle hochenergetischer elektromagnetischer Strahlung (5) emittierte hochenergetische Strahlung (8) mit einer Flächenleistungsdichte von 4,2 mW/cm$^2$ wird über einen Zeitraum von 10 Minuten zunächst durch einen Flächenlichtmodulator (SLM), dann zu 50% durch den zweiten Strahlteiler BS2, im Anschluss durch die zweite bikonvexen Linse (L2) und durch das Mikroskopobjektiv (MO1) in eine erste optische Faser des Bildwellenleiters (1, CFB) eingekoppelt. Der Flächenlichtmodulator (SLM) ist dabei so eingestellt, dass nur der Teil der hochenergetischen elektromagnetische Strahlung (8), welcher in die erste optische Faser einkoppelbar ist, durch den Flächenlichtmodulator (SLM) transmittiert wird. Der Teil der Strahlung (8) welche in Abwesenheit des Flächenlichtmodulators (SLM) in andere optische Fasern des Bildwellenleiters (1, CFB) eingekoppelt werden würde, wird vom Flächenlichtmodulator (SLM) absorbiert und/oder reflektiert. Im Anschluss wird die Laufzeit der ersten optischen Faser ermittelt und eine Vergrößerung des effektiven Brechungsindizes der ersten optischen Faser um einen Faktor in der Größenordnung von 0,001 ermittelt. Die Einstellung des Flächenlichtmodulators (SLM) wird im Anschluss so verändert, dass die hochenergetische elektromagnetische Strahlung in eine weitere optische Faser des Bildwellenleiters (1, CFB) eingekoppelt werden kann. Diese Konfiguration der Vorrichtung (3) wird in Fig. 8 schematsich dargestellt.

Anhand von Zeichnungen wird die Erfindung näher erläutert. Dabei zeigt

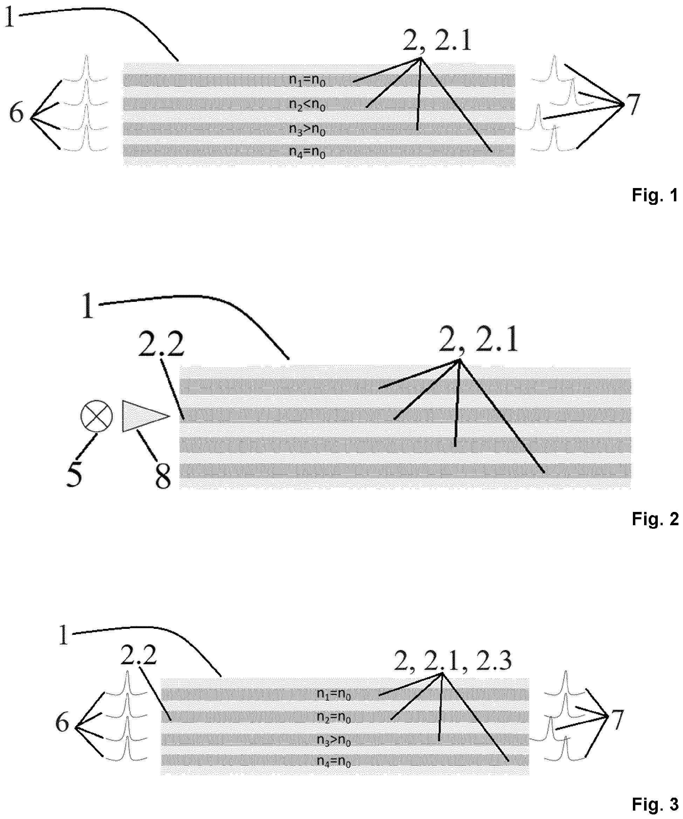

**Fig. 1** eine schematische Darstellung eines vier optische Fasern (2, 2.1) aufweisenden Bildwellenleiters (1), wobei die erste und vierte optische Faser einen effektiven Brechungsindex von $n_o$ aufweisen, die zweite optische Faser einen effektiven Brechungsindex $<n_o$ und die dritte optische Faser einen effektiven Brechungsindex $>n_o$. Ferner stellt Fig. 1 einen in alle optische Fasern des Bildwellenleiters (1) gleichzeitig eingekoppelten eintretenden Puls elektromagnetischer Strahlung (6) dar, welcher aufgrund von unterschiedlichen effektiven Brechungsindizes der optischen Fasern so als nicht gleichzeitig ausgekoppelter austretender Puls elektromagnetischer Strahlung (7) allen optischen Fasern Bildwellenleiters (1) ausgekoppelt wird, dass er die zweite optische Faser als erstes verlässt, daraufhin gleichzeitig die erste und vierte optische Faser verlässt und als letztes die dritte optische Faser verlässt. Die erste Teilmenge optischer Fasern (2.1) entspricht hier allen optischen Fasern (2) des Bildwellenleiters (1).

**Fig. 2** zeigt eine schematische Darstellung des vier optische Fasern (2, 2.1, 2.3) aufweisenden Bildwellenleiters (1) aus der **Fig. 1,** der mit hochenergetischer elektromagnetischer Strahlung (8) beaufschlagt wird um ein gewünschtes Laufzeitprofil zu erreichen. Das gewünschte Laufzeitprofil ist dadurch gekennzeichnet, dass die erste, die zweite, sowie die vierte optische Faser den gleichen effektiven Brechungsindex aufweisen und die dritte optische Faser einen höheren effektiven Brechungsindex aufweist, wodurch ein am ersten Ende des Bildwellenleiters (1) eingekoppelter Puls am zweiten Ende des Bildwellenleiters (1) aus der ersten, der zweiten, sowie der vierten optischen Faser gleichzeitig ausgekoppelt wird und aus der dritten optischen Faser verzögert. Da die dritte optische Faser bereits den höchsten effektiven Brechungsindex aufweist und die erste, sowie die vierte optische Faser den Medianwert der effektiven Brechungsindizes, wird der Brechungsindex der zweiten optischen Faser dem der ersten und vierten optischen Faser angeglichen. Deshalb wird die hochenergetische elektromagnetische Strahlung (8) einer Quelle (5) in die zweite optische Faser (2.2) eingekoppelt, um das gewünschte Laufzeitprofil der dritten Teilmenge optischer Fasern (2.3) des Bildwellenleiters (1) zu erreichen. Die zweite Teilmenge (2.2) umfasst dabei die zweite optische Faser und die dritte Teilmenge optischer Fasern (2.3) umfasst hier alle optischen Fasern (2) des Bildwellenleiters (1).

**Fig. 3** zeigt eine schematische Darstellung des vier optische Fasern (2, 2.1, 2.3) aufweisenden Bildwellenleiters (1) aus der **Fig. 2** nachdem die hochenergetische elektromagnetische Strahlung (8) in die zweite optische Faser (2.2) eingekoppelt wurde und einen gleichzeitig eingekoppelten eintretenden Puls elektromagnetischer Strahlung (6). Aufgrund der vorherigen Einkopplung hochenergetischer elektromagnetischer Strahlung (8) hat der effektive Brechungsindex der zweiten optischen Faser (2.2) den Wert $n_o$ erreicht und der austretende Puls elektromagnetischer Strahlung (7) weist das gewünschte Laufzeitprofil auf.

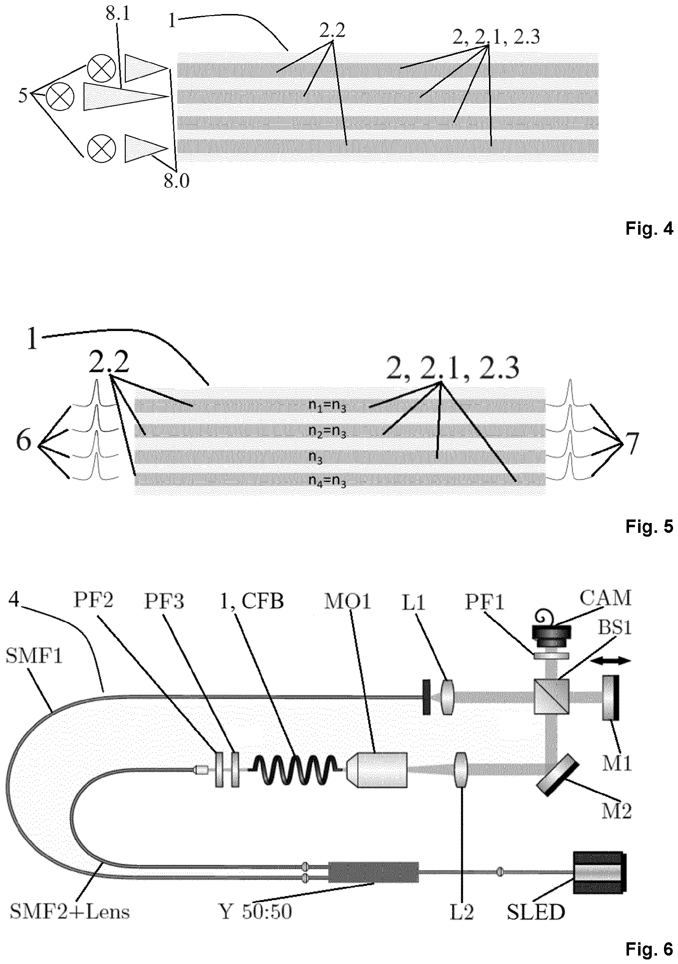

**Fig. 4** zeigt eine schematische Darstellung des vier optische Fasern (2, 2.1, 2.3) aufweisenden Bildwellenleiters (1) aus der **Fig. 1,** der mit hochenergetischer elektromagnetischer Strahlung (8.0, 8.1) beaufschlagt wird um die Laufzeitunterschiede der dritten Teilmenge optischer Fasern (2.3) des Bildwellenleiters (1) zu kompensieren. Da die dritte optische Faser bereits den höchsten effektiven Brechungsindex aufweist und die erste, die zweite und die vierte optische Faser einen geringeren effektiven Brechungsindizes aufweisen, wird der Brechungsindex der ersten, der zweiten und der vierten optischen Faser dem der dritten optischen Faser angeglichen. Deshalb wird die hochenergetische elektromagnetische Strahlung (8.0, 8.1) einer Quelle (5) in die zweite Teilmenge (2.2), umfassend die erste, die zweite und die vierte optische Faser (2.2) eingekoppelt. Da der effektive Brechungsindex der zweiten optischen Faser geringer ist, als der der ersten und vierten optischen Faser, weist die elektromagnetische Strahlung (8.1), die in die zweite optische Faser eingekoppelt wird, eine höhere Energie auf, als die in die erste und die dritte optische Faser eingekoppelte eine geringere Energie aufweisende elektromagnetische Strahlung (8.0), um die Kompensation der Laufzeitunterschiede des Bildwellenleiters (1) zu erreichen. Die Einkopplung der hochenergetischen elektromagnetischen Strahlung (8.0, 8.1) findet dabei in beliebiger zeitlicher Reihenfolge statt. Die dritte Teilmenge optischer Fasern (2.3) umfasst hier alle optischen Fasern (2) des Bildwellenleiters (1).

**Fig. 5** zeigt eine schematische Darstellung des vier optische Fasern (2, 2.1, 2.3) aufweisenden Bildwellenleiters (1) aus der **Fig. 4** nachdem die geringere Energie aufweisende hochenergetische elektromagnetische Strahlung (8.0) in die erste und vierte optische Faser (2.2) eingekoppelt wurde, sowie die höhere Energie aufweisende hochenergetische elektromagnetische Strahlung (8.1) in zweite optische Faser (2.2) eingekoppelt wurde und einen gleichzeitig eingekoppelten eintretenden Puls elektromagnetischer Strahlung (6). Aufgrund der vorherigen Einkopplung hochenergetischer elektromagnetischer Strahlung (8.0, 8.1) haben die effektiven Brechungsindizes der ersten, der zweiten und der vierten optischen Faser (2.2) den Wert des effektiven Brechungsindizes der dritten optischen Faser erreicht und die Laufzeitunterschiede des austretenden Pulses elektromagnetischer Strahlung (7) sind kompensiert.

**Fig. 6** zeigt eine schematische Darstellung der zum Vermessen des Laufzeitunterschiedes von Bildwellenleitern in mindestens einer Wellenlänge geeignete Anordnung (4), aufweisend eine Licht emittierende, eine Superlumineszenzdiode aufweisende Quelle elektromagnetischer Strahlung (SLED), einen Y-Wellenleiter (Y 50:50), einen ersten Lichtwellenleiter (SMF1), einen eine Linse aufweisenden zweiten Lichtwellenleiter (SMF2+Lens), eine erste bikonvexe Linse (L1), eine zweite bikonvexe Linse (L2), einen ersten Strahlteiler (BS1), einen ersten Spiegel (M1), einen zweiten Spiegel (M2), einen ersten Polarisationsfilter (PF1), einen zweiten Polarisationsfilter (PF2), einen dritten Polarisationsfilter (PF3), ein Mikroskopobjektiv (MO1) und einen eine Kamera aufweisenden bildgebenden Detektor für elektromagnetische Strahlung (CAM). Dabei wird Licht wird durch die Quelle elektromagnetischer Strahlung (SLED) emittiert und in den Y-Wellenleiter (Y 50:50) eingekoppelt und aufgeteilt. Eine erste Hälfte des Lichtes wird aus dem Y-Wellenleiter (Y 50:50) in den ersten Lichtwellenleiter (SMF1) eingekoppelt und so aus diesem ausgekoppelt, dass es zunächst auf die erste bikonvexe Linse (L1) trifft und von der ersten bikonvexen Linse (L1) so auf den ersten Strahlteiler (BS1) trifft, dass der erste Strahlteiler (BS1) 50 % der ersten Hälfte des Lichtes zum ersten Spiegel (M1) transmittiert, der erste Spiegel (M1) die 50 % der ersten Hälfte des Lichtes zum ersten Strahlteiler (BS1) reflektiert und der erste Strahlteiler (BS1) 50 % von den 50 % der ersten Hälfte des Lichtes durch den ersten Polarisationsfilter (PF1) zum eine Kamera aufweisenden bildgebenden Detektor für elektromagnetische Strahlung (CAM) reflektiert. Eine zweite Hälfte des Lichtes wird aus dem Y-Wellenleiter (Y 50:50) in das erste Ende des am zweiten Ende eine Linse aufweisenden zweiten Lichtwellenleiters (SMF2+Lens) eingekoppelt und am zweiten Ende aus diesem ausgekoppelt. Die zweite Hälfte des Lichtes gelangt anschließend durch den zweiten Polarisationsfilter (PF2), dann durch den dritten Polarisationsfilter (PF3) und wird daraufhin in einen als kohärentes Bündel optischer Fasern ausgebildeten Bildwellenleiter (1, CFB) gekoppelt. Die zweite Hälfte des Lichtes wird aus dem Bildwellenleiter (1, CFB) ausgekoppelt und gelangt durch das Mikroskopobjektiv (MO1) und anschließend die zweite bikonvexe Linse (L2) auf den zweiten Spiegel (M2), der es zum ersten Strahlteiler (BS1) reflektiert. Der erste Strahlteiler (BS1) transmittiert 50 % der zweiten Hälfte des Lichtes durch den ersten Polarisationsfilter (PF1) hindurch zum bildgebenden Detektor (CAM). Der erste Spiegel (M1) ist entlang der optischen Achse der 50 % der ersten Hälfte des Lichtes bewegbar angeordnet.

**Fig. 7** zeigt eine schematische Darstellung der Vorrichtung (3) zur Kompensation von Laufzeitunterschieden und/oder zur Implementation eines gewünschten Laufzeitprofils des Ausführungsbeispiels, aufweisend eine Licht emittierende, eine Superlumineszenzdiode aufweisende Quelle elektromagnetischer Strahlung (SLED), einen Y-Wellenleiter (Y 50:50), einen ersten Lichtwellenleiter (SMF1), einen eine Linse aufweisenden zweiten Lichtwellenleiter (SMF2+Lens), eine erste bikonvexe Linse (L1), eine zweite bikonvexe Linse (L2), einen ersten Strahlteiler (BS1), einen zweiten Strahlteiler (BS2), einen ersten Spiegel (M1), einen ersten Polarisationsfilter (PF1), einen zweiten Polarisationsfilter (PF2), einen dritten Polarisationsfilter (PF3), ein Mikroskopobjektiv (MO1), einen eine Kamera aufweisenden bildgebenden Detektor für elektromagnetische Strahlung (CAM), einen Flächenlichtmodulator (SLM) und eine Quelle hochenergetischer elektromagnetischer Strahlung (5). Dabei wird Licht wird durch die Quelle elektromagnetischer Strahlung (SLED) emittiert und in den Y-Wellenleiter (Y 50:50) eingekoppelt und aufgeteilt. Eine erste Hälfte des Lichtes wird aus dem Y-Wellenleiter (Y 50:50) in den ersten Lichtwellenleiter (SMF1) eingekoppelt und so aus diesem ausgekoppelt, dass es zunächst auf die erste bikonvexe Linse (L1) trifft und von der ersten bikonvexen Linse (L1) so auf den ersten Strahlteiler (BS1) trifft, dass der Strahlteiler (BS1) 50 % der ersten Hälfte des Lichtes zum ersten Spiegel (M1) transmittiert, der erste Spiegel (M1) die 50 % der ersten Hälfte des Lichtes zum Strahlteiler (BS1) reflektiert und der Strahlteiler (BS1) 50 % von den 50 % der ersten Hälfte des Lichtes durch den ersten Polarisationsfilter (PF1) zum eine Kamera aufweisenden bildgebenden Detektor für elektromagnetische Strahlung (CAM) reflektiert. Eine zweite Hälfte des Lichtes wird aus dem Y-Wellenleiter (Y 50:50) in das erste Ende des am zweiten Ende eine Linse aufweisenden zweiten Lichtwellenleiters (SMF2+Lens) eingekoppelt und am zweiten Ende aus diesem ausgekoppelt. Die zweite Hälfte des Lichtes gelangt anschließend durch den zweiten Polarisationsfilter (PF2), dann durch den dritten Polarisationsfilter (PF3) und wird daraufhin in einen als kohärentes Bündel optischer Fasern ausgebildeten Bildwellenleiter (1, CFB) gekoppelt. Die zweite Hälfte des Lichtes wird aus dem Bildwellenleiter (1, CFB) ausgekoppelt und gelangt durch das Mikroskopobjektiv (MO1) und anschließend die zweite bikonvexe Linse (L2) auf den zweiten Strahlteiler (BS2), der es zu 50% zum ersten Strahlteiler (BS1) reflektiert. Der Strahlteiler (BS1) transmittiert 50 % der 50% der zweiten Hälfte des Lichtes durch den ersten Polarisationsfilter (PF1) hindurch zum bildgebenden Detektor (CAM). Der erste Spiegel (M1) ist entlang der optischen Achse der 50 % der ersten Hälfte des Lichtes bewegbar angeordnet.

**Fig. 8** zeigt eine schematische Darstellung der Vorrichtung (3) zur Kompensation von Laufzeitunterschieden und/oder zur Implementation eines gewünschten Laufzeitprofils des Ausführungsbeispiels, Dabei wird hochenergetische elektromagnetische Strahlung (8) durch die Quelle (5) emittiert und gelangt zunächst durch den Flächenlichtmodulator (SLM), dann zu 50% durch den zweiten Strahlteiler BS2, im Anschluss durch die zweite bikonvexen Linse (L2) und durch das Mikroskopobjektiv (MO1) in eine erste optische Faser des Bildwellenleiters (1, CFB) eingekoppelt. Der Flächenlichtmodulator (SLM) ist dabei so eingestellt, dass nur der Teil der hochenergetischen elektromagnetische Strahlung (8), welcher in die erste optische Faser einkoppelbar ist, durch den Flächenlichtmodulator (SLM) transmittiert wird. Der Teil der Strahlung (8) welche in Abwesenheit des Flächenlichtmodulators (SLM) in andere optische Fasern des Bildwellenleiters (1, CFB) eingekoppelt werden würde, wird vom Flächenlichtmodulator (SLM) absorbiert und/oder reflektiert.

**Zitierte Nichtpatentliteratur:**

[1] Yoshinari Maezono et al., "Study of Refractive Index Change in Ge-Doped Fibers with Vacuum Ultraviolet Light Irradiation", 2008, Jpn. J. Appl. Phys. 47 7266

[2] Lancry et al., "Dependence of the femtosecond laser refractive index change thresholds on the chemical composition of doped-silica glasses", 2011, Opt. Mater. Express 1, 711-723

[3] Mirsky, S.K., Shaked, N.T., "Six-pack holography for dynamic profiling of thick and extended objects by simultaneous three-wavelength phase unwrapping with doubled field of view", 2023, Sei Rep 13, 19293

**Bezugszeichen**

| | |
|---|---|
| 1 | Bildwellenleiter |
| 2 | Optische Fasern |
| 2.1 | Erste Teilmenge optischer Fasern |
| 2.2 | Zweite Teilmenge optischer Fasern |
| 2.3 | Dritte Teilmenge optischer Fasern |
| 2.4 | Vierte Teilmenge optischer Fasern |
| 2.5 | Fünfte Teilmenge optischer Fasern |
| 3 | Vorrichtung zur Kompensation von Laufzeitunterschieden |
| 4 | Zum Vermessen des Laufzeitunterschiedes des Bildwellenleiters geeignete Anordnung |
| 5 | Quelle hochenergetischer elektromagnetischer Strahlung |
| 6 | Eintretender Puls elektromagnetischer Strahlung |
| 7 | Austretenden Puls elektromagnetischer Strahlung |
| 8 | Hochenergetische elektromagnetische Strahlung |
| 8.0 | Geringere Energie aufweisende hochenergetische elektromagnetische Strahlung |
| 8.1 | Höhere Energie aufweisende hochenergetische elektromagnetische Strahlung |
| SLED | Eine Superlumineszenzdiode aufweisende Quelle elektromagnetischer Strahlung |
| Y 50:50 | Y-Wellenleiter |
| SMF1 | Erster Lichtwellenleiter |
| SMF2+Lens | Eine Linse aufweisender zweiter Lichtwellenleiter |
| L1 | Erste bikonvexe Linse |
| L2 | Zweite bikonvexe Linse |
| BS1 | Erster Strahlteiler |
| BS2 | Zweiter Strahlteiler |
| M1 | Erster Spiegel |
| M2 | Zweiter Spiegel |
| PF1 | Erster Polarisationsfilter |
| PF2 | Zweiter Polarisationsfilter |
| PF3 | Dritter Polarisationsfilter |

MO1       Mikroskopobjektiv
CAM      Eine Kamera aufweisender bildgebender Detektor für elektromagnetische Strahlung
CFB       Kohärentes Bündel optischer Fasern
SLM       Flächenlichtmodulator

**Patentansprüche**

1. Verfahren zur Kompensation von Laufzeitunterschieden und/oder zur Implementation eines gewünschten Laufzeitprofils zumindest eines wenigstens zwei optische Fasern (2) aufweisenden Bildwellenleiters (1), umfassend die Schritte

   - Bereitstellen zumindest eines wenigstens zwei optische Fasern (2) aufweisenden Bildwellenleiters (1),
   - Auswählen einer ersten Teilmenge zumindest zweier optischer Fasern (2.1) des Bildwellenleiters (1) und Vermessen der Laufzeitunterschiede der optischen Fasern der ersten Teilmenge (2.1) in mindestens einer elektromagnetischen Wellenlänge,
   - Auswählen einer zweiten Teilmenge einer oder mehrerer optischer Fasern (2.2) und einer dritten Teilmenge mindestens zweier optischer Fasern (2.3) des Bildwellenleiters (1), Änderung des effektiven Brechungsindizes jeder der optischen Fasern der zweiten Teilmenge (2.2) durch longitudinale Einkopplung hochenergetischer elektromagnetischer Strahlung in jede der optischen Fasern der zweiten Teilmenge (2.2) an einem ersten Ende und/oder einem zweiten Ende des Bildwellenleiters (1), so, dass die Laufzeitunterschiede der dritten Teilmenge optischer Fasern (2.3) beliebig reduziert sind und/oder so, dass sich die Laufzeitunterschiede der dritten Teilmenge optischer Fasern (2.3) dem Wert des gewünschten Laufzeitprofils beliebig annähern,
   wobei die zweite Teilmenge optischer Fasern (2.2) mindestens eine optische Faser der ersten Teilmenge (2.1) umfasst und die dritte Teilmenge optischer Fasern (2.3) mindestens eine optische Faser der zweiten Teilmenge (2.2) umfasst und dabei die erste Teilmenge optischer Fasern (2.1) diese mindestens eine optische Faser der zweiten Teilmenge (2.2) ebenfalls umfasst,
   wobei die Durchführung des Schrittfolge ii) - iii) entweder einmalig erfolgt oder so oft erfolgt, bis eine gewünschte Kompensation der Laufzeitunterschiede und/oder das gewünschte Laufzeitprofil des zumindest einen Bildwellenleiters (1) in der mindestens einen Wellenlänge implementiert ist, wobei bei wiederholten Durchführungen der Schrittfolge ii) - iii) die Teilmengen optischer Fasern (2.1, 2.2 und 2.3) entweder jeweils den jeweiligen Teilmengen optischer Fasern (2.1, 2.2 und 2.3) der vorherigen Durchführung der Schrittfolge ii) - iii) gleichen oder neu ausgewählt werden und
   wobei die Schritte ii) und iii) sequenziell oder gleichzeitig erfolgen.

2. Verfahren nach Anspruch 1, **dadurch gekennzeichnet, dass** die hochenergetische elektromagnetische Strahlung ultra-kurze Pulse und/oder UV-Strahlung, insbesondere Femtosekunden-Laserpulse und/oder Excimer-Licht umfasst, wobei das Excimer-Licht bevorzugt 146-nm-Excimer-Licht oder 248-nm Excimer-Licht beinhaltet, wobei das Excimer-Licht der von Excimer-Lampen emittierbares Excimer-Licht und/oder Excimer-Laserlicht umfasst.

3. Verfahren nach Anspruch 1 oder 2, **dadurch gekennzeichnet, dass** eine gewünschte Änderung des effektiven Brechungsindex jeder der optischen Fasern der zweiten Teilmenge (2.2) dadurch erreicht wird, dass eine Modulation einer oder mehrerer Stellgrößen der hochenergetischen elektromagnetischen Strahlung, ausgewählt aus der Leistung, der Energie, der Pulsdauer, der Pulsform, dem spektralen Bereich, dem spektralen Verlauf der Leistung, dem zeitlichen Verlauf der Leistung, dem spektralen Verlauf der Energie, dem zeitlichen Verlauf der Energie und der Polarisation erfolgt.

4. Verfahren nach einem der vorhergehenden Ansprüche,

   - **dadurch gekennzeichnet, dass** der zumindest eine Bildwellenleiter (1) vor und/oder während der Durchführung der Schrittfolge ii)-iii) einer $H_2$ oder $N_2$ umfassenden Atmosphäre ausgesetzt ist, um den $H_2$- oder $N_2$-Partialdruck im inneren des Bildwellenleiters (1) zu erhöhen und/oder
   - **gekennzeichnet durch** Auswählen der Modulation des zeitlichen und spektralen Verlaufs der Strahlungsleistung von Pulsen der ultra-kurze Pulse aufweisenden hochenergetischen elektromagnetischen Strahlung vorteilhaft so, dass die über das gesamte Spektrum integrierte Strahlungsleistung bei einem ausgewählten Abstand vom ersten Ende des Bildwellenleiters (1) innerhalb mindestens einer der ausgewählten optischen Fasern einen Maximalwert annimmt und die Modulation des zeitlichen und spektralen Verlaufs der Strahlungsleistung der hochenergetischen elektromagnetischen Strahlung bei jeweils wiederholter Durchführung der Schrittfolge ii) - iii) besonders vorteilhaft so ausgewählt ist, dass bei jeder Wiederholung die Strahlungsleistung bei einem anderen Abstand vom ersten Ende des Bildwellenleiters als den bei der vorherigen Durchführung der Schrittfolge ii) - iii) ausgewählten Abstand einen Maximalwert annimmt.

5. Verfahren nach einem der vorhergehenden Ansprüche, **dadurch gekennzeichnet, dass** das Verfahren dazu geeignet ist, durch ultrakurze Pulse hochenergetischer elektromagnetischer Strahlung verursachbare Schäden des Bildwellenleiters (1) im Bereich des ersten Endes und/oder des zweiten Endes gering zu halten

   - durch Verringern der Differenz zwischen den effektiven Brechungsindizes der Fasern und des an dem ersten Ende und/oder dem zweiten Ende des Bildwellenleiters (1) angrenzenden Mediums während der Durchführung des Verfahrens durch

      ∘ Umgeben des ersten Endes und/oder dem zweiten Ende des Bildwellenleiters (1) mit einer Immersionsflüssigkeit, bevorzugt einem Immersionsöl und/oder
      ∘ Bringen in Berührungskontakt zumindest einer Glasplatte mit dem ersten Ende und/oder dem zweiten Ende des Bildwellenleiters (1),
      wobei die Materialien, aus dem die Immersionsflüssigkeit und/oder die Glasplatte bestehen, mindestens jeweils ein Material umfassen, dessen Brechungsindex dem effektiven Brechungsindex mindestens einer optischen Faser der zweiten Teilmenge ausgewählter optischer Fasern (2.2) beliebig nah ist und/oder

   - durch Entfernen eines Teils des zumindest einen Bildwellenleiters (1) entlang einer Ebene am ersten Ende und/oder am zweiten Ende nach der Durchführung von Schritt iii), wobei die Ebene senkrecht zur optischen Achse des Bildwellenleiters (1) ist und die Länge des zu entfernenden Teils oder der zu entfernenden Teile des Bildwellenleiters (1) entlang der optischen Achse der Länge des Teils oder der Teile des Bildwellenleiters (1) entspricht oder entsprechen, der oder die durch eine Absorption zumindest eines Teils der hochenergetischen elektromagnetischer Strahlung beschädigt wurde oder wurden und/oder
   - durch ein Aufweiten der Kerne der optischen Fasern am ersten Ende und/oder am zweiten Ende des Bildwellenleiters (1).

6. Verfahren nach einem der vorhergehenden Ansprüche, **dadurch gekennzeichnet, dass** das Vermessen des Laufzeitunterschiedes mittels Weißlichtinterferometrie und/oder OCT und/oder Mehrwellenlängenholographie erfolgt.

7. Verfahren nach einem der vorhergehenden Ansprüche, **dadurch gekennzeichnet, dass** nach Durchführung von Schritt iii) ein Verfahren zur Kompensation von Phasenstörung von mindestens zwei Wellenlängen $\lambda_k$ des zumindest einen Bildwellenleiters (1) und/oder zur Implementation mindestens einer optischen Funktion, welche Ausbreitungsrichtungen elektromagnetischer Strahlung von mindestens einer Wellenlänge $\lambda_f$ beim Ein- und/oder Austritt in den und/oder aus dem Bildwellenleiter (1) ändert erfolgt, umfassend das Modulieren der einen funktionalen Zusammenhang mit einer Referenzweglänge aufweisenden elektromagnetischen Phasenstörung $\varphi_{ist}$ einer fünften Teilmenge mindestens einer optischen Faser j (2.5), die ausgewählt ist aus einer vierten Teilmenge zweier oder mehrerer optischer Fasern (2.4) des Bildwellenleiters (1), für jede der Wellenlängen $\lambda_k$ und/oder $\lambda_f$, umfassend die Teilschritte

   a) Vermessen der elektromagnetischen Phasenstörung $\varphi_{ist}$ für jede der Wellenlängen $\lambda_k$ und/oder $\lambda_f$ an den optischen Fasern (2.4) der vierten Teilmenge,
   b) Bestimmung einer gewünschten modulierten Phase $\varphi_{soll}$ für jede der fünften Teilmenge ausgewählter optischer Fasern j (2.5) und für jede der Wellenlängen λk und/oder λf, wobei die gewünschte modulierte Phase $\varphi_{soll}$ für jede der Wellenlängen λk und/oder λf unabhängig voneinander bestimmt wird oder abhängig von $\varphi_{soll}$ für eine oder mehrere der anderen Wellenlängen λk und/oder λf bestimmt wird,
   c) Ermittlung eines funktionalen Zusammenhangs zwischen einer Stellgröße $x_j$ und einer Phasenänderung $\varphi_{stell}$ für jede der Wellenlängen λk und/oder λf und jeder der ausgewählten optischen Fasern j der fünften Teilmenge (2.5),
   d) Definition einer Fehlerfunktion $f$ zur Beschreibung der Gesamtabweichung zwischen einer resultierenden Phase $\varphi_{res} = (\varphi_{ist} + \varphi_{stell}) \mod(2\pi)$ und der gewünschten modulierten Phase $\varphi_{soll}$ über alle Wellenlängen λk und/oder λf für jede der ausgewählten optischen Fasern j der fünften Teilmenge (2.5),
   e) Ermittlung des Wertes $x_{j\_fmin}$ der Stellgröße $x_j$ bei der die Fehlerfunktion $f$ einen minimalen Wert annimmt für jede der ausgewählten optischen Fasern $j$ der fünften
   f) Teilmenge (2.5),

      - Bereitstellen und Positionieren eines Elements zur Kompensation von Phasenstörung von mindestens

zwei Wellenlängen $\lambda_k$ eines Bildwellenleiters (1) und/oder zur Implementation mindestens einer optischen Funktion, welche Ausbreitungsrichtungen elektromagnetischer Strahlung von mindestens einer Wellenlänge $\lambda_f$ beim Ein- und/oder Austritt in den und/oder aus dem Bildwellenleiter (1) ändert, hinter dem ersten Ende und/oder hinter dem zweiten Ende des Bildwellenleiters (1), derart, dass das Element entlang der optischen Achse jeder der ausgewählten optischen Fasern $j$ der fünften Teilmenge (2.5).den Wert $x_{j\_fmin}$ der Stellgröße $x_j$ aufweist,
und/oder

- Verkürzung und/oder Verlängerung jeder ausgewählten optischen Fasern $j$ der fünften Teilmenge (2.5) zur Kompensation der Phasenstörung und/oder zur Implementation einer Funktion, welche Ausbreitungsrichtungen elektromagnetischer Strahlung beim Ein- und/oder Austritt in den und/oder aus dem Bildwellenleiter (1) ändert, am ersten Ende und/oder am zweiten Ende des Bildwellenleiters (1), derart, dass die Verkürzung und/oder die Verlängerung für jede der ausgewählten optischen Fasern $j$ der fünften Teilmenge (2.5).und jeder der Wellenlängen $\lambda_k$ und/oder $\lambda_f$ den Wert $x_{j\_fmin}$ der Stellgröße $x_j$ aufweist,

so dass der das Element und/oder die Verkürzung und/oder Verlängerung jeder der ausgewählten optischen Fasern $j$ der fünften Teilmenge (2.5) aufweisende Bildwellenleiter (1) für jede der Wellenlängen $\lambda_k$ und/oder $\lambda_f$ und jeder der ausgewählten optischen Fasern $j$ der fünften Teilmenge (2.5) eine resultierende Phase $\varphi_{res\_fmin}$ aufweist, bei der die Fehlerfunktion $f$ einen minimalen Wert annimmt.

8. Vorrichtung (3) zur Kompensation von Laufzeitunterschieden und/oder zur Implementation eines gewünschten Laufzeitprofils zumindest eines wenigstens zwei optische Fasern (2) aufweisenden Bildwellenleiters (1), umfassend eine zum Vermessen des Laufzeitunterschiedes von Bildwellenleitern in mindestens einer Wellenlänge geeignete Anordnung (4), wobei

- die zum Vermessen des Laufzeitunterschiedes des Bildwellenleiters (1) geeignete Anordnung (4) eine zur Änderung der effektiven Brechungsindizes optischer Fasern geeignete Quelle hochenergetischer elektromagnetischer Strahlung (5) umfasst und die Quelle (5)

  ◦ als Strahlungsquelle für das Vermessen des Laufzeitunterschiedes der optischen Fasern und einer gleichzeitigen Änderung des effektiven Brechungsindizes der optischen Fasern verwendbar ist und/oder
  ◦ durch Herunterregeln der Strahlungsleistung und/oder durch die Implementation eines optischen Filters zwischen der Quelle (5) und dem Bildwellenleiter (1) als Quelle niederenergetischer Strahlung betreibbar und für das Vermessen des Laufzeitunterschiedes der optischen Fasern verwendbar ist
  und/oder

- die Vorrichtung (3) eine von der Anordnung (4) separate zur Änderung der effektiven Brechungsindizes optischer Fasern geeignete Quelle (5) hochenergetischer Strahlung umfasst,

ferner umfassend mindestens eine erste Positionierungsvorrichtung, die dazu geeignet ist

- den Bildwellenleiter (1) und die Anordnung (4) so zueinander zu positionieren, um das Vermessen der Laufzeitunterschiedes und/oder die Änderung der effektiven Brechungsindizes von optischen Fasern des Bildwellenleiters (1) zu ermöglichen und/oder
- den Bildwellenleiter (1) und die Quelle hochenergetischer elektromagnetischer Strahlung (5) so zueinander zu positionieren, dass die longitudinale Einkopplung von durch die Quelle (5) emittierbarer Strahlung in mindestens eine optische Faser ermöglicht ist.

9. Vorrichtung (3) nach Anspruch 8, **dadurch gekennzeichnet, dass** die Quelle hochenergetischer elektromagnetischer Strahlung mindestens einen ultra-kurz gepulsten Laser, und/oder mindestens eine UV-Lichtquelle, insbesondere mindestens einen Femtosekundenlaser und/oder mindestens eine Excimer-Lichtquelle umfasst, wobei die Excimer-Lichtquelle bevorzugt eine 146-nm-Excimer-Lichtquelle oder eine 248-nm-Excimer-Lichtquelle ist und wobei die Excimer-Lichtquelle mindestens eine Excimerlampe und/oder mindestens einen Excimerlaser umfasst.

10. Vorrichtung (3) nach Anspruch 8 oder 9, **dadurch gekennzeichnet, dass** die Quelle hochenergetischer elektromagnetischer Strahlung zu einer Modulation einer oder mehrerer Stellgrößen der Strahlung, ausgewählt aus der der Leistung, der Energie, der Pulsdauer, der Pulsform, dem spektralen Bereich, dem spektralen Verlauf der Leistung, dem zeitlichen Verlauf der Leistung, dem spektralen Verlauf der Energie, dem zeitlichen Verlauf der Energie und der Polarisation ausgebildet ist.

**11.** Vorrichtung (3) nach einem der Ansprüche 8 bis 10,

- **dadurch gekennzeichnet, dass** sie mindestens eine $H_2$- und/oder $N_2$-Kammer aufweist, wobei die $H_2$- und/oder $N_2$-Kammer einen luftdicht verschließbaren Gasbehälter und eine an den Gasbehälter anschließbare, zum Leiten von $H_2$- und/oder $N_2$-Gas geeignete Leitung umfasst, wobei die Leitung an ein $H_2$- und/oder $N_2$-Gasnetz anschließbar ist und/oder an einem zum Beinhalten von $H_2$- und/oder $N_2$-Gas geeigneten Druckbehälter anschließbar ist und bevorzugt ein zum Herausbefördern des im Gasbehälter befindlichen Gases geeignetes Gerät und/oder ein zum Hereinbefördern des $H_2$- und/oder $N_2$-Gases geeignetes Gerät umfasst und wobei die Kammer dazu ausgebildet ist, den zumindest einen Bildwellenleiter (1) zu beinhalten und die Kammer vorteilhaft mindestens einen für zumindest die Halbwertsbreite

  ◦ der zum Vermessen des Laufzeitunterschiedes verwendbare Strahlung und
  ◦ der hochenergetischen Strahlung
  transparenten Bereich aufweist und die erste Positionierungsvorrichtung in der Kammer angeordnet ist oder die Kammer mindestens eine zweite Positionierungsvorrichtung aufweist, die dazu ausgebildet ist, den Bildwellenleiter (1) so innerhalb der Kammer zu positionieren, dass die hochenergetische Strahlung und die zum Vermessen des Laufzeitunterschiedes verwendbare Strahlung in den Bildwellenleiter (1) longitudinal einkoppelbar ist und/oder

- **dadurch gekennzeichnet, dass** die Modulation des zeitlichen und spektralen Verlaufs der Strahlungsleistung des ultra-kurz gepulsten Lasers vorteilhaft so ausbildbar ist, dass die über das gesamte Spektrum integrierte Strahlungsleistung bei longitudinaler Einkopplung der Strahlung in die mindestens eine optische Faser des Bildwellenleiters (1) an einem auswählbaren Abstand vom ersten Ende des Bildwellenleiters (1) innerhalb der mindestens einen optischen Faser einen Maximalwert annimmt.

**12.** Vorrichtung (3) nach einem der Ansprüche 8 bis 11, **dadurch gekennzeichnet, dass**

- die Vorrichtung (3) mindestens eine Apparatur aufweist, welche dazu ausgebildet ist, die Kerne der optischen Fasern des Bildwellenleiters (1) am ersten Ende und/oder am zweiten Ende aufzuweiten und/oder
- die Vorrichtung (3) mindestens einen für das Beinhalten einer Immersionsflüssigkeit, insbesondere eines Immersionsöls geeigneten Flüssigkeitsbehälter aufweist, der dazu ausgebildet ist, zumindest das erste Ende und/oder zumindest das zweite Ende des zumindest einen Bildwellenleiters (1) zu beinhalten und mindestens einen für zumindest die Halbwertsbreite des Spektralbereichs

  ◦ der zum Vermessen der Laufzeitunterschiede verwendbare Strahlung und
  ◦ der hochenergetischen Strahlung
  transparenten Bereich aufweist und die erste Positionierungsvorrichtung im Flüssigkeitsbehälter angeordnet ist oder der Flüssigkeitsbehälter mindestens eine dritte Positionierungsvorrichtung aufweist, die dazu ausgebildet ist, zumindest das erste Ende und/oder zumindest das zweite Ende des Bildwellenleiters (1) so innerhalb der Kammer zu positionieren, dass die zum Vermessen des Laufzeitunterschiedes verwendbare Strahlung und die hochenergetischen Strahlung in den Bildwellenleiter (1) longitudinal einkoppelbar ist und/oder

- die Vorrichtung (3) mindestens eine Glasplatte aufweist, wobei die Glasplatte und/oder der Bildwellenleiter (1) so positionierbar sind/ist, dass die Glasplatte in Berührungskontakt mit dem ersten Ende und/oder dem zweiten Ende des Bildwellenleiters (1) steht,

wobei die Materialien, aus dem die Immersionsflüssigkeit und/oder die Glasplatte bestehen, mindestens jeweils ein Material umfassen, dessen Brechungsindex dem effektiven Brechungsindex mindestens einer optischen Faser des Bildwellenleiters (1) beliebig nah ist und das jeweilige Material zumindest für die Halbwertsbreite der Wellenlänge der elektromagnetischen Strahlung transparent ist, die von der zum Vermessen des Laufzeitunterschiedes von Bildwellenleitern geeigneten Anordnung (4) emittierbar und absorbierbar ist.

**13.** Vorrichtung (3) nach einem der Ansprüche 8 bis 12, **dadurch gekennzeichnet, dass** die zum Vermessen des Laufzeitunterschiedes von Bildwellenleitern in mindestens einer Wellenlänge geeignete Anordnung (4) mindestens ein Weißlichtinterferometer und/oder mindestens einen optischen Kohärenztomografen umfasst.

**14.** Vorrichtung (3) nach einem der Ansprüche 8 bis 13, **dadurch gekennzeichnet, dass** sie eine Einrichtung zur Kompensation von elektromagnetischer Phasenstörung von mindestens zwei Wellenlängen $\lambda_k$ des zumindest einen Bildwellenleiters (1) und/oder zur Implementation einer Funktion, welche Ausbreitungsrichtungen elektromagnetischer Strahlung von mindestens einer Wellenlänge $\lambda_f$ beim Ein- und/oder Austritt in den und/oder aus dem Bildwellenleiter (1) ändert aufweist, umfassend eine zum Vermessen der Phasenstörung von Bildwellenleitern in mindestens zwei Wellenlängen geeignete Anordnung (4), ferner umfassend

- ein Element, welches zur Kompensation von elektromagnetischer Phasenstörung von mindestens zwei Wellenlängen $\lambda_k$ und/oder zur Implementation einer Funktion, welche Ausbreitungsrichtungen elektromagnetischer Strahlung von mindestens einer Wellenlänge $\lambda_f$ beim Ein- und/oder Austritt in den und/oder aus dem Bildwellenleiter (1) ändert geeignet ist, wobei das Element an einem ersten Ende und/oder einem zweiten Ende des Bildwellenleiters (1) positionierbar ist und derart moduliert oder modulierbar ist, dass das Element entlang der elektromagnetischen Ausbreitungsrichtung eines oder mehrerer ausgewählter Wellenleiter eine Stellgröße $x_{j\_fmin}$ aufweist und/oder
- ein zur Verkürzung und/oder Verlängerung optischer Fasern von Bildwellenleitern geeignetes Gerät, wobei das Gerät, wobei der Bildwellenleiter (1) und das Gerät so zueinander positionierbar sind, dass eine Verkürzung und/oder Verlängerung von optischen Fasern des Bildwellenleiters (1) möglich, so, dass einer Verkürzung und/oder Verlängerung unterzogene optische Fasern eine Stellgröße $x_{j\_fmin}$ aufweisen,

wobei die Stellgröße $x_{j\_fmin}$ mittels Durchführung der Teilschritte a) bis f) des Verfahrens nach Anspruch 7 ermittelbar ist.

15. Verwendung des Verfahrens nach einem der Ansprüche 1 bis 7 und/oder einer Vorrichtung (3) nach einem der Ansprüche 8 bis 14 zur Kompensation von Laufzeitunterschieden und/oder zur Implementation eines gewünschten Laufzeitprofils eines mindestens zwei optische Fasern aufweisenden Bildwellenleiters in der Krebsdiagnostik, der nichtlinearen Endomikroskopie, der OCT, der Swept-Source-OCT, zur ungestörten Übertragung von Femtosekunden-Pulsen und/oder zur Korrektur von Laufzeitunterschieden, die in Bildwellenleitern auftreten, welche miteinander verdrillte optische Fasern aufweisen.

$n_1 = n_0$
$n_2 < n_0$
$n_3 > n_0$
$n_4 = n_0$

2, 2.1

Fig. 1

2, 2.1

2.2

Fig. 2

2.2

2, 2.1, 2.3

$n_1 = n_0$
$n_2 = n_0$
$n_3 > n_0$
$n_4 = n_0$

Fig. 3

Fig. 4

Fig. 5

Fig. 6

Fig. 7

Fig. 8

**Europäisches Patentamt**
**European Patent Office**
**Office européen des brevets**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2018/011309 A1 (ANDRESEN ESBEN [FR] ET AL) 11. Januar 2018 (2018-01-11) | 1-3, 5-10, 12-15 | INV. G02B6/06 |
| Y | * Absatz [0056] - Absatz [0096] * <br> * Abbildungen 2-10 * | 4,11 | |
| | ----- | | |
| Y | EP 1 258 754 A2 (FUJIKURA LTD [JP]) 20. November 2002 (2002-11-20) | 4,11 | |
| A | * Absatz [0007] - Absatz [0050] * <br> * Abbildungen 1, 2 * | 1-3, 5-10, 12-15 | |
| | ----- | | |
| A,D | US 2021/382290 A1 (RIGNEAULT HERVÉ [FR] ET AL) 9. Dezember 2021 (2021-12-09) <br> * Absatz [0077] - Absatz [0137] * <br> * Abbildung 10 * | 1-15 | |
| | ----- | | |
| A | ESBEN RAVN ANDRESEN ET AL: "Measurement and compensation of residual group delay in a multi-core fiber for lensless endoscopy", JOURNAL OF THE OPTICAL SOCIETY OF AMERICA - B., Bd. 32, Nr. 6, 26. Mai 2015 (2015-05-26), Seiten 1221-1228, XP055537517, US ISSN: 0740-3224, DOI: 10.1364/JOSAB.32.001221 * das ganze Dokument * | 1-15 | **RECHERCHIERTE SACHGEBIETE (IPC)** <br><br> G02B |
| | ----- | | |

Der vorliegende Recherchenbericht wurde für alle Patentansprüche erstellt

| Recherchenort | Abschlußdatum der Recherche | Prüfer |
|---|---|---|
| Den Haag | 12. September 2024 | Szachowicz, Marta |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..............................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 24 16 4836

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-09-2024

| Im Recherchenbericht angeführtes Patentdokument | Datum der Veröffentlichung | Mitglied(er) der Patentfamilie | Datum der Veröffentlichung |
|---|---|---|---|
| US 2018011309 A1 | 11-01-2018 | EP 3234666 A1 | 25-10-2017 |
| | | FR 3030956 A1 | 24-06-2016 |
| | | JP 6720183 B2 | 08-07-2020 |
| | | JP 2018502638 A | 01-02-2018 |
| | | US 2018011309 A1 | 11-01-2018 |
| | | WO 2016097191 A1 | 23-06-2016 |
| EP 1258754 A2 | 20-11-2002 | DE 60215072 T2 | 05-04-2007 |
| | | EP 1258754 A2 | 20-11-2002 |
| | | US 2002172479 A1 | 21-11-2002 |
| | | US 2005238302 A1 | 27-10-2005 |
| US 2021382290 A1 | 09-12-2021 | EP 3853655 A1 | 28-07-2021 |
| | | FR 3086398 A1 | 27-03-2020 |
| | | JP 7430711 B2 | 13-02-2024 |
| | | JP 2022502124 A | 11-01-2022 |
| | | US 2021382290 A1 | 09-12-2021 |
| | | WO 2020058043 A1 | 26-03-2020 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20210382290 A1

- US 20220248938 A1

**In der Beschreibung aufgeführte Nicht-Patentliteratur**

- **YOSHINARI MAEZONO et al.** Study of Refractive Index Change in Ge-Doped Fibers with Vacuum Ultraviolet Light Irradiation. *Jpn. J. Appl. Phys.*, 2008, vol. 47, 7266
- **LANCRY et al.** Dependence of the femtosecond laser refractive index change thresholds on the chemical composition of doped-silica glasses. *Opt. Mater. Express*, 2011, vol. 1, 711-723

- **MIRSKY, S.K** ; **SHAKED, N.T**. Six-pack holography for dynamic profiling of thick and extended objects by simultaneous three-wavelength phase unwrapping with doubled field of view. *Sei Rep*, 2023, vol. 13, 19293